# EUROPEAN PATENT SPECIFICATION

(11) **EP 2 152 702 B1**
(45) Date of publication and mention of the grant of the patent: **24.08.2011**
(21) Application number: 08737246.2
(22) Date of filing: 02.05.2008
(51) Int. Cl.: C07D 417/12, C07D 417/14, A61K 31/506, A61P 35/00

(54) **AMINO-THIAZOLYL- PYRIMIDINE DERIVATIVES AND THEIR USE FOR THE TREATMENT OF CANCER**
AMINOTHIAZOLYLPYRIMIDINDERIVATE UND IHRE VERWENDUNG BEI DER BEHANDLUNG VON KREBS
DÉRIVÉS D'AMINO-THIAZOLYL-PYRIMIDINE ET LEUR UTILISATION POUR LE TRAITEMENT DU CANCER

(30) Priority: 04.05.2007 US 915966 P
(43) Date of publication of application: 17.02.2010
(73) Proprietor: AstraZeneca AB, 151 85 Södertälje (SE)
(72) Inventor: ALMEIDA, Lynsie, Waltham, Massachusetts 02451 (US); GUAN, Huiping, Waltham, Massachusetts 02451 (US); IOANNIDIS, Stephanos, Waltham, Massachusetts 02451 (US); LAMB, Michelle, Waltham, Massachusetts 02451 (US); PENG, Bo, Waltham, Massachusetts 02451 (US)
(86) International application number: PCT/GB2008/050322
(87) International publication number: WO 2008/135786

(56) References cited:
- WO-A-2006/115452
- WO-A-2006/123113
- WO-A-2007/049041

## Description

### Field of the Invention

The present invention relates to novel compounds, their pharmaceutical compositions and methods of use. In addition, the present invention relates to therapeutic methods for the treatment and prevention of cancers and to the use of these compounds in the manufacture of medicaments for the treatment and prevention of myeloproliferative disorders and cancers.

### Background of the Invention

The JAK (Janus-associated kinase)/STAT (signal transducers and activators of transcription) signalling pathway is involved in a variety of hyperproliferative and cancer related processes including cell-cycle progression, apoptosis, angiogenesis, invasion, metastasis and evasion of the immune system (Haura et al., Nature Clinical Practice Oncology, 2005, 2(6), 315-324; Verna et al., Cancer and Metastasis Reviews, 2003, 22, 423-434).

The JAK family consists of four non-receptor tyrosine kinases Tyk2, JAK1, JAK2, and JAK3, which play a critical role in cytokine- and growth factor mediated signal transduction. Cytokine and/or growth factor binding to cell-surface receptor(s), promotes receptor dimerization and facilitates activation of receptor-associated JAK by autophosphorylation. Activated JAK phosphorylates the receptor, creating docking sites for SH2 domain-containing signalling proteins, in particular the STAT family of proteins (STAT1, 2, 3, 4, 5a, 5b and 6). Receptor-bound STATs are themselves phosphorylated by JAKs, promoting their dissociation from the receptor, and subsequent dimerization and translocation to the nucleus. Once in the nucleus, the STATs bind DNA and cooperate with other transcription factors to regulate expression of a number of genes including, but not limited to, genes encoding apoptosis inhibitors (e.g. Bcl-XL, Mcl-1) and cell cycle regulators (e.g. Cyclin D1/D2, c-myc) (Haura et al., Nature Clinical Practice Oncology, 2005, 2(6), 315-324; Verna et al., Cancer and Metastasis Reviews, 2003, 22, 423-434).

Over the past decade, a considerable amount of scientific literature linking constitutive JAK and/or STAT signalling with hyperproliferative disorders and cancer has been published. Constitutive activation of the STAT family, in particular STAT3 and STAT5, has been detected in a wide range of cancers and hyperproliferative disorders (Haura et al., Nature Clinical Practice Oncology, 2005, 2(6), 315-324). Furthermore, aberrant activation of the JAK/STAT pathway provides an important proliferative and/or anti-apoptotic drive downstream of many kinases (e.g. Flt3, EGFR) whose constitutive activation have been implicated as key drivers in a variety of cancers and hyperproliferative disorders (Tibes et al., Annu Rev Pharmacol Toxicol 2550, 45, 357-384; Choudhary et al., International Journal of Hematology 2005, 82(2), 93-99; Sordella et al., Science 2004, 305, 1163-1167). In addition, impairment of negative regulatory proteins, such as the suppressors of cytokine signalling (SOCS) proteins, can also influence the activation status of the JAK/STAT signalling pathway in disease (JC Tan and Rabkin R, Pediatric Nephrology 2005, 20, 567-575).

Several mutated forms of JAK2 have been identified in a variety of disease settings. For example, translocations resulting in the fusion of the JAK2 kinase domain with an oligomerization domain, TEL-JAK2, Bcr-JAK2 and PCM1-JAK2, have been implicated in the pathogenesis of various hematologic malignancies (SD Turner and Alesander DR, Leukemia, 2006, 20, 572-582). More recently, a unique acquired mutation encoding a valine-to-phenylalanine (V617F) substitution in JAK2 was detected in a significant number of polycythemia vera, essential thrombocythemia and idiopathic myelofibrosis patients and to a lesser extent in several other diseases. The mutant JAK2 protein is able to activate downstream signalling in the absence of cytokine stimulation, resulting in autonomous growth and/or hypersensitivity to cytokines and is believed to play a critical role in driving these diseases (MJ Percy and McMullin MF, Hematological Oncology 2005, 23(3-4), 91-93).

JAKs (in particular JAK3) play an important biological roles in the immunosuppressive field and there are reports of using JAK kinase inhibitors as tools to prevent organ transplant rejections (Changelian, P.S. et al, Science, 2003, 302, 875-878). Merck (Thompson, J. E. et al Bioorg. Med. Chem. Lett. 2002, 12, 1219-1223) and Incyte (WO2005/105814) reported imidazole based JAK2/3 inhibitors with enzyme potency at single nM levels. Vertex PCT publications have described azaindoles as JAK inhibitors (WO2005/95400). AstraZeneca has published quinoline-3-carboxamides as JAK3 inhibitors (WO2002/92571).

In addition to the above, Vertex Pharmaceuticals has described pyrazole compounds as inhibitors of GSK3, Aurora, etc. in WO2002/50065, WO2002/62789, WO2003/027111 and WO2004/37814; and AstraZeneca have reported pyrazole compounds as inhibitors against IGF-1 receptor kinase - WO2003/48133 - and Trk in WO2005/049033, WO2005/103010, WO2006/082392. WO 2007/04 9041, WO 2006/123113 and WO 2006/11 5452 all disclose aminopyrazolyl pyrimidine derivatives for the treatment of cancer.

### Summary of the Invention

In accordance with the present invention, applicants have hereby discovered compounds of Formula (I): 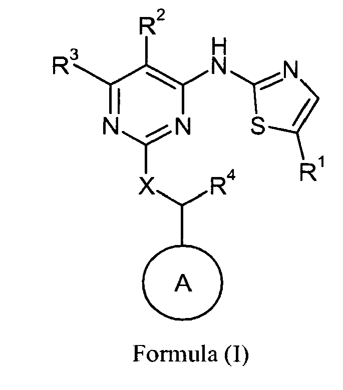 or pharmaceutically acceptable salts thereof.

The compounds of Formula (I) are believed to possess JAK kinase inhibitory activity and are accordingly useful for their anti-proliferation and/or pro-apoptotic activity and in methods of treatment of the human or animal body. The invention also relates to processes for the manufacture of said compound, or pharmaceutically acceptable salts thereof, to pharmaceutical compositions containing it and to its use in the manufacture of medicaments for use in the production of an anti-proliferation and/or pro-apoptotic effect in warm-blooded animals such as man. Also in accordance with the present invention the applicants provide methods of using said compound, or pharmaceutically acceptable salts thereof, in the treatment of myeloproliferative disorders, myelodysplastic syndrome and cancer.

The properties of the compounds of Formula (I) are expected to be of value in the treatment of myeloproliferative disorders, myelodysplastic syndrome, and cancer by inhibiting the tyrosine kinases, particularly the JAK family and more particularly JAK2. Methods of treatment target tyrosine kinase activity, particularly the JAK family activity and more particularly JAK2 activity, which is involved in a variety of myeloproliferative disorders, myelodysplastic syndrome and cancer related processes. Thus, inhibitors of tyrosine kinases, particularly the JAK family and more particularly JAK2, are expected to be active against myeloproliferative disorders such as chronic myeloid leukemia, polycythemia vera, essential thrombocythemia, myeloid metaplasia with myelofibrosis, idiopathic myelofibrosis, chronic myelomonocytic leukemia and hypereosinophilic syndrome, myelodysplastic syndromes and neoplastic disease such as carcinoma of the breast, ovary, lung, colon, prostate or other tissues, as well as leukemias, myelomas and lymphomas, tumors of the central and peripheral nervous system, and other tumor types such as melanoma, fibrosarcoma and osteosarcoma. Tyrosine kinase inhibitors, particularly the JAK family inhibitors and more particularly JAK2 inhibitors are also expected to be useful for the treatment other proliferative diseases including but not limited to autoimmune, inflammatory, neurological, and cardiovascular diseases.

Furthermore, the compounds of Formula (I), or pharmaceutically acceptable salts thereof, are expected to be of value in the treatment or prophylaxis of against myeloproliferative disorders selected from chronic myeloid leukemia, polycythemia vera, essential thrombocythemia, myeloid metaplasia with myelofibrosis, idiopathic myelofibrosis, chronic myelomonocytic leukemia and hypereosinophilic syndrome, myelodysplastic syndromes and cancers selected from oesophageal cancer, myeloma, hepatocellular, pancreatic, cervical cancer, Ewings sarcoma, neuroblastoma, Kaposi's sarcoma, ovarian cancer, breast cancer, colorectal cancer, prostate cancer, bladder cancer, melanoma, lung cancer - non small cell lung cancer (NSCLC), and small cell lung cancer (SCLC), gastric cancer, head and neck cancer, mesothelioma, renal cancer, lymphoma and leukaemia; particularly myeloma, leukemia, ovarian cancer, breast cancer and prostate cancer.

### Detailed Description of the Invention

The present invention relates to compounds of Formula (I): 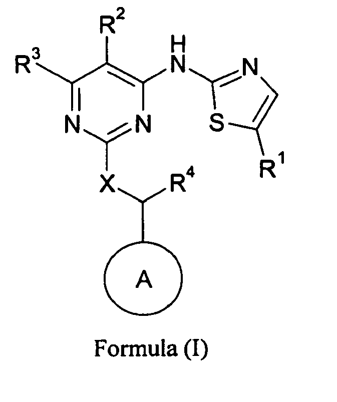 or pharmaceutically acceptable salts thereof, wherein:
**Ring A** is selected from 4-fluorophenyl, 5-fluoropyridin-2-yl, and 5-fluoropyrimidin-2-yl;
**X** is -NH-;
**R¹** is selected from -CN, methyl, and -C(O)₂Me;
**R²** is selected from H, fluoro, and chloro;
**R³** is selected from H, methoxy, 4-methylpiperazin-1-yl, morpholin-4-yl, and piperazin-1-yl; and
**R⁴** is methyl.

In this specification the prefix C_{x-y} as used in terms such as C_{x-y}alkyl and the like (where x and y are integers) indicates the numerical range of carbon atoms that are present in the group; for example, C₁₋₄alkyl includes C₁alkyl (methyl), C₂alkyl (ethyl), C₃alkyl (propyl and isopropyl) and C₄alkyl (butyl, 1-methylpropyl, 2-methylpropyl, and *t-*butyl).

Alkyl - As used herein the term "alkyl" refers to both straight and branched chain saturated hydrocarbon radicals having the specified number of carbon atoms. References to individual alkyl groups such as "propyl" are specific for the straight chain version only and references to individual branched chain alkyl groups such as 'isopropyl', are specific for the branched chain version only.

Alkenyl - As used herein, the term "alkenyl" refers to both straight and branched chain hydrocarbon radicals having the specified number of carbon atoms and containing at least one carbon-carbon double bond. For example, "C₂₋₆alkenyl" includes, but is not limited to, groups such as C₂₋₆alkenyl, C₂₋₄alkenyl, ethenyl, 2-propenyl, 2-methyl-2-propenyl, 3-butenyl, 4-pentenyl, and 5-hexenyl.

Alkynyl - As used herein, the term "alkynyl" refers to both straight and branched chain hydrocarbon radicals having the specified number of carbon atoms and containing at least one carbon-carbon triple bond. For example, "C₂₋₆alkynyl" includes, but is not limited to, groups such as C₂₋₆alkynyl, C₂₋₄alkynyl, ethynyl, 2-propynyl, 2-methyl-2-propynyl, 3-butynyl, 4-pentynyl, and 5-hexynyl.

Halo - As used herein, the term "halo" refers to fluoro, chloro, bromo and iodo. In one aspect, the term "halo" may refer to fluoro, chloro, and bromo. In another aspect, the term "halo" may refer to fluoro and chloro.

Carbocyclyl - As used herein, the term "carbocyclyl" refers to a saturated, partially saturated, or unsaturated, mono or bicyclic carbon ring that contains 3 to 12 ring atoms, of which one or more -CH₂- groups may be optionally replaced with a corresponding number of -C(O)- groups. Illustrative examples of "carbocyclyl" include, but are not limited to, adamantyl, cyclopropyl, cyclobutyl, cyclopentyl, cyclopentenyl, cyclohexyl, cyclohexenyl, indanyl, naphthyl, oxocyclopentyl, 1-oxoindanyl, phenyl, and tetralinyl. In one aspect, "carbocyclyl" may refer to cyclopropyl.

Heterocyclyl - As used herein, the term "heterocyclyl" refers to a saturated, partially saturated, or unsaturated, mono or bicyclic ring containing 4 to 12 ring atoms of which at least one ring atom is selected from nitrogen, sulfur, and oxygen, and which may, unless otherwise specified, be carbon or nitrogen linked, and of which a -CH₂- group can optionally be replaced by a -C(O)-. Ring sulfur atoms may be optionally oxidized to form S-oxides. Ring nitrogen atoms may be optionally oxidized to form N-oxides. Illustrative examples of the term "heterocyclyl" include, but are not limited to, 1,3-benzodioxolyl, 3,5-dioxopiperidinyl, furanyl, imidazolyl, indolyl, isoquinolinyl, isothiazolyl, isoxazolyl, morpholino, 2-oxa-5-azabicyclo[2.2.1]hept-5-yl, oxazolyl, 2-oxopyrrolidinyl, 2-oxo-1,3-thiazolidinyl, piperazinyl, piperidyl, 2*H-*pyranyl, pyrazolyl, pyridinyl, pyrrolyl, pyrrolidinyl, pyrrolidinyl, pyrimidinyl, pyrazinyl, pyrazolyl, pyridazinyl, 4-pyridonyl, quinolyl, tetrahydrofuranyl, tetrahydropyranyl, thiazolyl, thiadiazolyl, thiazolidinyl, thiomorpholino, thiophenyl, pyridine-*N*-oxidyl and quinoline-*N*-oxidyl.

5- or 6-Membered Heterocyclyl - In one aspect, "heterocyclyl" may be "5- or 6-membered heterocyclyl," which refers to a saturated, partially saturated, or unsaturated, monocyclic ring containing 5 or 6 ring atoms, of which at least one ring atom is selected from nitrogen, sulfur, and oxygen, and of which a -CH₂- group may be optionally replaced by a -C(O)- group. Unless otherwise specified, "5- or 6-membered heterocyclyl" groups may be carbon or nitrogen linked. Ring nitrogen atoms may be optionally oxidized to form an N-oxide. Ring sulfur atoms may be

optionally oxidized to form S-oxides. Illustrative examples of "5- or 6-membered heterocyclyl" include, but are not limited to, 3,5-dioxopiperidinyl, furanyl, imidazolyl, isothiazolyl, isoxazolyl, morpholino, oxazolyl, 2-oxopyrrolidinyl, 2-oxo-1,3-thiazolidinyl, piperazinyl, piperidyl, 2*H-*pyranyl, pyrazolyl, pyridinyl, pyrrolyl, pyrrolidinyl, pyrrolidinyl, pyrimidinyl, pyrazinyl, pyrazolyl, pyridazinyl, 4-pyridonyl, tetrahydrofuranyl, tetrahydropyranyl, thiazolyl, thiadiazolyl, thiazolidinyl, thiomorpholino, thiophenyl, pyridine-*N*-oxidyl.

6-Membered Heterocyclyl - In another aspect, "heterocyclyl" and "5- or 6-membered heterocyclyl" may be "6-membered heterocyclyl," which refers to a saturated, partially saturated, or unsaturated, monocyclic ring containing 6 ring atoms, of which at least one ring atom is selected from nitrogen, sulfur, and oxygen, and of which a -CH₂- group may be optionally replaced by a -C(O)- group. Unless otherwise specified, "6-membered heterocyclyl" groups may be carbon or nitrogen linked. Ring nitrogen atoms may be optionally oxidized to form an N-oxide. Ring sulfur atoms may be optionally oxidized to form S-oxides. Illustrative examples of "6-membered heterocyclyl" include, but are not limited to, 3,5-dioxopiperidinyl, morpholino, piperazinyl, piperidinyl, 2*H-*pyranyl, pyrazinyl, pyridazinyl, pyridinyl, and pyrimidinyl.

6-Membered Heteroaryl - In still another aspect, "heterocyclyl", "5- or 6-membered heterocyclyl," and "6-membered heterocyclyl" may be "6-membered heteroaryl." The term "6-membered heteroaryl" is intended to refer to a monocyclic, aromatic heterocyclyl ring containing 6 ring atoms. Unless otherwise specified, "6-membered heteroaryl" groups are carbon linked. Illustrative examples of the term "6-membered heteroaryl" include, but are not limited to, pyrazinyl, pyridazinyl, pyrimidinyl, and pyridinyl.

Where a particular R group (e.g. R^{1a}, R¹⁰, etc.) is present in a compound of Formula (I) more than once, it is intended that each selection for that R group is independent at each occurrence of any selection at any other occurrence. For example, the -N(R)₂ group is intended to encompass: 1) those -N(R)₂ groups in which both R substituents are the same, such as those in which both R substituent are, for example, C₁₋₆alkyl; and 2) those -N(R)₂ groups in which each R substituent is different, such as those in which one R substituent is, for example, H, and the other R substituent is, for example, carbocyclyl.

Unless specifically stated, the bonding atom of a group may be any suitable atom of that group; for example, propyl includes prop-1-yl and prop-2-yl.

Effective Amount - As used herein, the phrase "effective amount" means an amount of a compound or composition which is sufficient enough to significantly and positively modify the symptoms and/or conditions to be treated (e.g., provide a positive clinical response). The effective amount of an active ingredient for use in a pharmaceutical composition will vary with the particular condition being treated, the severity of the condition, the duration of the treatment, the nature of concurrent therapy, the particular active ingredient(s) being employed, the particular pharmaceutically-acceptable excipient(s)/carrier(s) utilized, and like factors within the knowledge and expertise of the attending physician.

In particular, an effective amount of a compound of Formula (I) for use in the treatment of cancer is an amount sufficient to symptomatically relieve in a warm-blooded animal such as man, the symptoms of cancer and myeloproliferative diseases, to slow the progression of cancer and myeloproliferative diseases, or to reduce in patients with symptoms of cancer and myeloproliferative diseases the risk of getting worse.

Leaving Group - As used herein, the phrase "leaving group" is intended to refer to groups readily displaceable by a nucleophile such as an amine nucleophile, and alcohol nucleophile, or a thiol nucleophile. Examples of suitable leaving groups include halo, such as chloro and bromo, and sulfonyloxy group, such as methanesulfonyloxy and toluene-4-sulfonyloxy.

Optionally substituted - As used herein, the phrase "optionally substituted," indicates that substitution is optional and therefore it is possible for the designated group to be either substituted or unsubstituted. In the event a substitution is desired, any number of hydrogens on the designated group may be replaced with a selection from the indicated substituents, provided that the normal valency of the atoms on a particular substituent is not exceeded, and that the substitution results in a stable compound.

One or More - In one aspect, when a particular group is designated as being optionally substituted with "one or more" substituents, the particular may be unsubstituted. In another aspect, the particular group may bear one substituent. In another aspect, the particular substituent may bear two substituents. In still another aspect, the particular group may bear three substituents. In yet another aspect, the particular group may bear four substituents. In a further aspect, the particular group may bear one or two substituents. In still a further aspect, the particular group may be unsubstituted, or may bear one or two substituents.

Pharmaceutically Acceptable - As used herein, the term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Protecting Group - As used herein, the term "protecting group" is intended to refer to those groups used to prevent selected reactive groups (such as carboxy, amino, hydroxy, and mercapto groups) from undergoing undesired reactions.

Illustrative examples of suitable protecting groups for a hydroxy group include, but are not limited to, an acyl group; alkanoyl groups such as acetyl; aroyl groups, such as benzoyl; silyl groups, such as trimethylsilyl; and arylmethyl groups, such as benzyl. The deprotection conditions for the above hydroxy protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively a silyl group such as trimethylsilyl may be removed, for example, by fluoride or by aqueous acid; or an arylmethyl group such as a benzyl group may be removed, for example, by hydrogenation in the presence of a catalyst such as palladium-on-carbon.

Illustrative examples of suitable protecting groups for an amino group include, but are not limited to, acyl groups; alkanoyl groups such as acetyl; alkoxycarbonyl groups, such as methoxycarbonyl, ethoxycarbonyl, and *t-*butoxycarbonyl; arylmethoxycarbonyl groups, such as benzyloxycarbonyl; and aroyl groups, such benzoyl. The deprotection conditions for the above amino protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a *t-*butoxycarbonyl group may be removed, for example, by treatment with a suitable acid as hydrochloric, sulfuric, phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid, for example boron trichloride). A suitable alternative protecting group for a primary amino group is, for example, a phthaloyl group, which may be removed by treatment with an alkylamine, for example dimethylaminopropylamine or 2-hydroxyethylamine, or with hydrazine. Another suitable protecting group for an amine is, for example, a cyclic ether such as tetrahydrofuran, which may be removed by treatment with a suitable acid such as trifluoroacetic acid.

The protecting groups may be removed at any convenient stage in the synthesis using conventional techniques well known in the chemical art, or they may be removed during a later reaction step or work-up.

The compounds discussed herein in many instances were named and/or checked with ACD/Name by ACD/Labs®.

Compounds of Formula (I) may form stable pharmaceutically acceptable acid or base salts, and in such cases administration of a compound as a salt may be appropriate. Examples of acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethyl-sulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate. Examples of base salts include ammonium salts; alkali metal salts such as sodium, lithium and potassium salts; alkaline earth metal salts such as aluminum, calcium and magnesium salts; salts with organic bases such as dicyclohexylamine salts and N-methyl-D-glucamine; and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others. Non-toxic physiologically-acceptable salts are preferred, although other salts may be useful, such as in isolating or purifying the product.

The salts may be formed by conventional means, such as by reacting the free base form of the product with one or more equivalents of the appropriate acid in a solvent or medium in which the salt is insoluble, or in a solvent such as water, which is removed *in vacuo* or by freeze drying or by exchanging the anions of an existing salt for another anion on a suitable ion-exchange resin.

Some compounds of Formula (I) may have chiral centres and/or geometric isomeric centres (E-and Z- isomers), and it is to be understood that the invention encompasses all such optical, diastereoisomers and geometric isomers. The invention further relates to any and all tautomeric forms of the compounds of Formula (I).

It is also to be understood that certain compounds of Formula (I) can exist in solvated as well as unsolvated forms such as, for example, hydrated forms. It is to be understood that the invention encompasses all such solvated forms.

Additional embodiments of the invention are as follows. These additional embodiments relate to compounds of Formula (I) and pharmaceutically acceptable salts thereof. Such specific substituents may be used, where appropriate, with any of the definitions, claims or embodiments defined hereinbefore or hereinafter.

### Ring A

In a further aspect, **Ring A** may be selected from 4-fluorophenyl, 5-fluoropyridin-2-yl, and 5-fluoropyrimidin-2-yl.

In still a further aspect, **Ring A** may be 4-fluorophenyl.

In yet a further aspect, **Ring A** may be 5-fluoropyridin-2-yl.

In one aspect, **Ring A** may be 5-fluoropyrimidin-2-yl.

**X**

In another aspect, **X** may be -NH-.

**R¹**

In still a further aspect, **R¹** may be methyl.

In yet a further aspect, **R¹** may be -C(O)₂Me.

In yet a further aspect, **R¹** may be selected from -CN, methyl, and -C(O)₂Me.

**R²**

In still another aspect, **R²** may be selected from H.

In a further aspect, **R²** may be selected from H, fluoro, and chloro.

In still a further aspect, **R²** may be selected from fluoro.

In yet a further aspect, **R²** may be selected from chloro.

**R³**

In a further aspect, **R³** may be selected from H, 4-methylpiperazin-1-yl, morpholin-4-yl, and piperazin-1-yl.

In still a further aspect, **R³** may be selected from H, methoxy, 4-methylpiperazin-1-yl, morpholin-4-yl, and piperazin-1-yl.

In one aspect, **R³** may be H.

In another aspect, **R³** may be morpholin-4-yl.

In still another aspect, **R³** may be 4-methylpiperazin-1-yl.

In a further aspect, **R³** may be piperazin-1-yl.

**R⁴**

In still another aspect, **R⁴** may be methyl.

In yet another aspect, the compound of Formula (I) may be a compound of Formula (Ia): 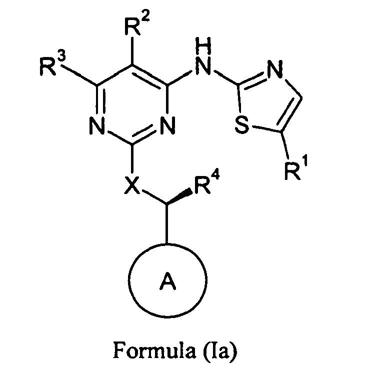 or a pharmaceutically acceptable salt thereof, wherein Ring A, X, R¹, R², R³, and R⁴ are as defined hereinabove.

**Ring A, X, R¹, R², R³**, and **R⁴**

In still another aspect, **Ring A** may be selected from 4-fluorophenyl, 5-fluoropyridin-2-yl, and 5-fluoropyrimidin-2-yl;

**R¹** may be selected from -CN, methyl, and -C(O)₂Me;

**R²** may be selected from H, fluoro, and chloro;

**R³** may be selected from H, 4-methylpiperazin-1-yl, morpholin-4-yl, and piperazin-1-yl; and

**R⁴** may be methyl.

In still another aspect, there is provided a compound selected from
5-Chloro-*N*²-[(1*S*)-1-(4-fluorophenyl)ethyl]-*N*⁴-(5-methyl-1,3-thiazol-2-yl)pyrimidine-2,4-diamine;
Methyl 2-[(5-chloro-2-{[(1*S*)-1-(4-fluorophenyl)ethyl]amino}pyrimidin-4-yl)amino]-1,3-thiazole-5-carboxylate;
5-Chloro-*N*²-[(1*S*)-1-(5-fluoropyridin-2-yl)ethyl]-*N*⁴-(5-methyl-1,3-thiazol-2-yl)pyrimidine-2,4-diamine;
5-Fluoro-*N*²-[(1*S*)-1-(5-fluoropyrimidin-2-yl)ethyl]-*N*⁴-(5-methyl-1,3-thiazol-2-yl)-6-morpholin-4-ylpyrimidine-2,4-diamine;
N²-[(S)-1-(5-Fluoro-pyridin-2-yl)-ethyl]-6-(4-methyl-piperazin-1-yl)-N⁴-(5-methyl-thiazol-2-yl)-pyrimidine-2,4-diamine;
*N*²-[(1*S*)-1-(5-Fluoropyrimidin-2-yl)ethyl]-*N*⁴-(5-methyl-1,3-thiazol-2-yl)-6-morpholin-4-ylpyrimidine-2,4-diamine;
*N*²-[(1*S*)-1-(5-Fluoropyridin-2-yl)ethyl]-*N*⁴-(5-methyl-1,3-thiazol-2-yl)-6-morpholin-4-ylpyrimidine-2,4-diamine;
2-[(2-{[(1*S*)-1-(5-Fluoropyridin-2-yl)ethyl]amino}-6-morpholin-4-ylpyrimidin-4-yl)amino]-1,3-thiazole-5-carbonitrile;
2-[(2-{[(1*S*)-1-(5-Fluoropyrimidin-2-yl)ethyl]amino}-6-morpholin-4-ylpyrimidin-4-yl)amino]-1,3-thiazole-5-carbonitrile; and
(*S*)-*N²*-(1-(5-Fluoropyrimidin-2-yl)ethyl)-6-methoxy-*N⁴*-(5-methylthiazol-2-yl)pyrimidine-2,4-diamine.

### Utility

### JAK2

The compounds of Formula (I) have utility for the treatment of myeloproliferative disorders, myelodysplastic syndrome and cancer by inhibiting the JAK tyrosine kinases, particularly the JAK2 family. Methods of treatment target tyrosine kinase activity, particularly the JAK family activity and more particularly JAK2 activity, which is involved in a variety of myeloproliferative disorders, myelodysplastic syndrome and cancer related processes. Thus, inhibitors of tyrosine kinase, particularly the JAK family and more particularly JAK2, are expected to be active against myeloproliferative disorders such as chronic myeloid leukemia, polycythemia vera, essential thrombocythemia, myeloid metaplasia with myelofibrosis, idiopathic myelofibrosis, chronic myelomonocytic leukemia and hypereosinophilic syndrome, myelodysplastic syndromes and neoplastic disease such as carcinoma of the breast, ovary, lung, colon, prostate or other tissues, as well as leukemias, myelomas and lymphomas, tumors of the central and peripheral nervous system, and other tumor types such as melanoma, fibrosarcoma and osteosarcoma. Tyrosine kinase inhibitors, particularly the JAK family inhibitors and more particularly JAK2 inhibitors are also expected to be useful for the treatment other proliferative diseases including but not limited to autoimmune, inflammatory, neurological, and cardiovascular diseases.

The compounds of Formula (I) have been shown to inhibit tyrosine kinases, particularly the JAK family and more particularly JAK2, as determined by the JAK2 Assay described herein.

The compounds of Formula (I) should also be useful as standards and reagents in determining the ability of a potential pharmaceutical to inhibit tyrosine kinases, particularly the JAK family and more particularly JAK2. These would be provided in commercial kits comprising a compound of this invention.

JAK2 kinase activity was determined by measuring the kinase's ability to phosphorylate synthetic tyrosine residues within a generic polypeptide substrate using an Amplified Luminescent Proximity Assay (Alphascreen) technology (PerkinElmer, 549 Albany Street, Boston, MA).

To measure JAK2 kinase activity, a commercially available purified enzyme may be used. The enzyme may be C-terminal His6-tagged, recombinant, human JAK2, amino acids 808-end, (Genbank Accession number NM 004972) expressed by baculovirus in Sf21 cells (Upstate Biotechnology MA). After incubation of the kinase with a biotinylated substrate and adenosine triphosphate (ATP) for 60 minutes at room temperature, the kinase reaction may be stopped by the addition of 30 mM ethylenediaminetetraacetic acid (EDTA). The reaction may be performed in 384 well microtitre plates and the reaction products amy be detected with the addition of streptavidin coated Donor Beads and phosphotyrosine-specific antibodies coated Acceptor Beads using the EnVision Multilabel Plate Reader after an overnight incubation at room temperature.

| | |
|---|---|
| Peptide substrate | TYK2 (Tyr 1054/1055 biotinylated peptide) Cell Signalling Technology #2200B. 402µM stock. |
| ATP Km | 30 µM |
| Assay conditions | 150pM JAK2 enzyme, 30 µM ATP, 80nM Tyk2, 10mM MgCl₂, 50mM Hepes buffer pH 7.5, 1mM DTT, 0.025% Tween 20 |
| Incubation | 60 minutes, room temperature |
| Termination/Detection conditions | 6.3mM HEPES, 30 mM EDTA, 525 µg/ml BSA, 40 mM NaCl, 0.007%triton® X-100, 12 ng/ml of Donor Beads, 12 ng/ml of Acceptor Beads |
| Detection incubation | overnight, room temperature |
| Fluometer settings | Excitation = 680 nm Emission = 570 nm Excitation Time = 180 ms Total Measurement Time=550 ms |

Although the pharmacological properties of the compounds of the Formula (I) may vary with structural change, it is believed that in general, activity possessed by compounds of the Formula (I) may be demonstrated at IC₅₀ concentrations (concentrations to achieve 50% inhibition) or doses at a level below 10 µM.

When tested in an in-vivo assay based on the assay described above, the JAK inhibitory activity of the following examples was measured at the following IC₅₀S.

| **Ex** | **IC₅₀ (µM)** |
|---|---|
| 1 | 0.006 |
| 2 | 0.170 |
| 3 | 0.902 |
| 4 | 0.003 |
| 5 | 0.003 |
| 6 | 0.003 |
| 7 | 0.003 |
| 8 | 0.003 |
| 10 | 0.003 |

Thus, in one aspect, there is provided a compound of Formula (I), or a pharmaceutically acceptable salt thereof, for use as a medicament.

In another aspect, there is provided the use of a compound of Formula (I), or a pharmaceutically acceptable salt thereof, in the manufacture of a medicament for the treatment or prophylaxis of myeloproliferative disorders, myelodysplastic syndrome, and cancer, in a warm-blooded animal such as man.

In still another aspect, there is provided the use of a compound of Formula (I), or a pharmaceutically acceptable salt thereof, in the manufacture of a medicament for the treatment or prophylaxis of myeloproliferative disorders, myelodysplastic syndrome and cancers (solid and hematologic tumors), fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acromegaly, acute and chronic inflammation, bone diseases, and ocular diseases with retinal vessel proliferation, in a warm-blooded animal such as man.

In yet another aspect, there is provided the use of a compound of Formula (I), or a pharmaceutically acceptable salt thereof, in the manufacture of a medicament for treating chronic myeloid leukemia, polycythemia vera, essential thrombocythemia, myeloid metaplasia with myelofibrosis, idiopathic myelofibrosis, chronic myelomonocytic leukemia and hypereosinophilic syndrome, myelodysplastic syndromes and cancers selected from oesophageal cancer, myeloma, hepatocellular, pancreatic, cervical cancer, Ewings sarcoma, neuroblastoma, Kaposi's sarcoma, ovarian cancer, breast cancer, colorectal cancer, prostate cancer, bladder cancer, melanoma, lung cancer - non small cell lung cancer (NSCLC), and small cell lung cancer (SCLC), gastric cancer, head and neck cancer, mesothelioma, renal cancer, lymphoma and leukaemia, in a warm-blooded animal such as man.

In a further aspect, there is provided the use of a compound of Formula (I), or a pharmaceutically acceptable salt thereof, in the manufacture of a medicament for the production of an anti-proliferative effect, in a warm-blooded animal such as man.

In yet a further aspect, there is provided the use of a compound of Formula (I), or a pharmaceutically acceptable salt thereof, in the manufacture of a medicament for the treatment of cancer.

In yet a further aspect, there is provided a compound of Formula (I), or a pharmaceutically acceptable salt thereof, for use in treating myeloproliferative disorders, myelodysplastic syndrome, and cancer, in a warm-blooded animal such as man.

In one aspect, there is provided a compound of Formula (I), or a pharmaceutically acceptable salt thereof, for use in treating myeloproliferative disorders, myelodysplastic syndrome, and cancers (solid and hematologic tumors), fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acromegaly, acute and chronic inflammation, bone diseases, and ocular diseases with retinal vessel proliferation, in a warm-blooded animal such as man.

In another aspect, there is provided a compound of Formula (I), or a pharmaceutically acceptable salt thereof, for use in the treating chronic myeloid leukemia, polycythemia vera, essential thrombocythemia, myeloid metaplasia with myelofibrosis, idiopathic myelofibrosis, chronic myelomonocytic leukemia and hypereosinophilic syndrome, myelodysplastic syndromes and cancers selected from oesophageal cancer, myeloma, hepatocellular, pancreatic, cervical cancer, Ewings sarcoma, neuroblastoma, Kaposi's sarcoma, ovarian cancer, breast cancer, colorectal cancer, prostate cancer, bladder cancer, melanoma, lung cancer - non small cell lung cancer (NSCLC), and small cell lung cancer (SCLC), gastric cancer, head and neck cancer, mesothelioma, renal cancer, lymphoma and leukaemia, in a warm-blooded animal such as man.

In still another aspect, there is provided a compound of Formula (I), or a pharmaceutically acceptable salt thereof, for use in the production of an anti-proliferative effect, in a warm-blooded animal such as man.

In a further aspect, there is provided a compound of Formula (I), or a pharmaceutically acceptable salt thereof, for use in the treatment of cancer in a warm-blooded animal such as man.

In still a further aspect, where reference is made to the treatment (or prophylaxis) of cancer, it may particularly refer to the treatment (or prophylaxis) of mesoblastic nephroma, mesothelioma, acute myeloblastic leukemia, acute lymphocytic leukemia, multiple myeloma, oesophageal cancer, myeloma, hepatocellular, pancreatic, cervical cancer, Ewings sarcoma, neuroblastoma, Kaposi's sarcoma, ovarian cancer, breast cancer including secretory breast cancer, colorectal cancer, prostate cancer including hormone refractory prostate cancer, bladder cancer, melanoma, lung cancer - non small cell lung cancer (NSCLC), and small cell lung cancer (SCLC), gastric cancer, head and neck cancer, renal cancer, lymphoma, thyroid cancer including papillary thyroid cancer, mesothelioma, leukaemia, tumors of the central and peripheral nervous system, melanoma, fibrosarcoma including congenital fibrosarcoma and osteosarcoma. More particularly it refers to prostate cancer. In addition, more particularly it refers to SCLC, NSCLC, colorectal cancer, ovarian cancer and / or breast cancer. In a further aspect it may refer to hormone refractory prostate cancer.

In yet a further aspect, there is provided a pharmaceutical composition comprising a compound of Formula (I), or a pharmaceutically acceptable salt thereof, and at least one pharmaceutically acceptable carrier, diluent, or excipient.

In one aspect, there is provided a pharmaceutical composition comprising a compound of Formula (I), or a pharmaceutically acceptable salt thereof, and at least one pharmaceutically acceptable carrier, diluent, or excipient.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients well known in the art. Thus, compositions intended for oral use may contain, for example, one or more coloring, sweetening, flavoring and/or preservative agents.

Suitable pharmaceutically acceptable excipients for a tablet formulation include, for example, inert diluents such as lactose, sodium carbonate, calcium phosphate or calcium carbonate; granulating and disintegrating agents such as corn starch or algenic acid; binding agents such as starch; lubricating agents such as magnesium stearate, stearic acid or talc; preservative agents such as ethyl or propyl p-hydroxybenzoate; and anti-oxidants, such as ascorbic acid. Tablet formulations may be uncoated or coated either to modify their disintegration and the subsequent absorption of the active ingredient within the gastrointestinal tract, or to improve their stability and/or appearance, in either case, using conventional coating agents and procedures well known in the art.

Compositions for oral use may be in the form of hard gelatin capsules in which the active ingredient is mixed with an inert solid diluent, for example, calcium carbonate, calcium phosphate or kaolin, or as soft gelatin capsules in which the active ingredient is mixed with water or an oil such as peanut oil, liquid paraffin, or olive oil.

Aqueous suspensions generally contain the active ingredient in finely powdered form or in the form of nano or micronized particles together with one or more suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives such as ethyl or propyl p-hydroxybenzoate; anti-oxidants such as ascorbic acid); coloring agents; flavoring agents; and/or sweetening agents such as sucrose, saccharine or aspartame.

Oily suspensions may be formulated by suspending the active ingredient in a vegetable oil such as arachis oil, olive oil, sesame oil or coconut oil or in a mineral oil such as liquid paraffin. The oily suspensions may also contain a thickening agent such as beeswax, hard paraffin or cetyl alcohol. Sweetening agents such as those set out above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water generally contain the active ingredient together with a dispersing or wetting agent, suspending agent and one or more preservatives. Suitable dispersing or wetting agents and suspending agents are exemplified by those already mentioned above. Additional excipients such as sweetening, flavoring and coloring agents, may also be present.

The pharmaceutical compositions of the invention may also be in the form of oil-in-water emulsions. The oily phase may be a vegetable oil, such as olive oil or arachis oil, or a mineral oil, such as for example liquid paraffin or a mixture of any of these. Suitable emulsifying agents may be, for example, naturally-occurnng gums such as gum acacia or gum tragacanth, naturally-occurring phosphatides such as soya bean, lecithin, an esters or partial esters derived from fatty acids and hexitol anhydrides (for example sorbitan monooleate) and condensation products of the said partial esters with ethylene oxide such as polyoxyethylene sorbitan monooleate. The emulsions may also contain sweetening, flavoring and preservative agents.

Syrups and elixirs may be formulated with sweetening agents such as glycerol, propylene glycol, sorbitol, aspartame or sucrose, and may also contain a demulcent, preservative, flavoring and/or coloring agent.

The pharmaceutical compositions may also be in the form of a sterile injectable aqueous or oily suspension, which may be formulated according to known procedures using one or more of the appropriate dispersing or wetting agents and suspending agents, which have been mentioned above. A sterile injectable preparation may also be a sterile injectable solution or suspension in a non-toxic parenterally-acceptable diluent or solvent, for example a solution in 1,3-butanediol.

Compositions for administration by inhalation may be in the form of a conventional pressurized aerosol arranged to dispense the active ingredient either as an aerosol containing finely divided solid or liquid droplets. Conventional aerosol propellants such as volatile fluorinated hydrocarbons or hydrocarbons may be used and the aerosol device is conveniently arranged to dispense a metered quantity of active ingredient.

For further information on formulation the reader is referred to Chapter 25.2 in Volume 5 of Comprehensive Medicinal Chemistry (Corwin Hansch; Chairman of Editorial Board), Pergamon Press 1990.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 4 g of active agent compounded with an appropriate and convenient amount of excipients which may vary from about 5 to about 98 percent by weight of the total composition. Dosage unit forms will generally contain about 1 mg to about 500 mg of an active ingredient. For further information on Routes of Administration and Dosage Regimes the reader is referred to Chapter 25.3 in Volume 5 of Comprehensive Medicinal Chemistry (Corwin Hansch; Chairman of Editorial Board), Pergamon Press 1990.

As stated above the size of the dose required for the therapeutic or prophylactic treatment of a particular disease state will necessarily be varied depending on the host treated, the route of administration and the severity of the illness being treated. Preferably a daily dose in the range of 1-50 mg/kg is employed. Accordingly, the optimum dosage may be determined by the practitioner who is treating any particular patient.

The anti-cancer treatment defined herein may be applied as a sole therapy or may involve, in addition to the compound of the invention, conventional surgery or radiotherapy or chemotherapy. Such chemotherapy may include one or more of the following categories of anti-tumor agents:
- (i): antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines including 5-fluorouracil and tegafur, raltitrexed, methotrexate, cytosine arabinoside and hydroxyurea); antitumor antibiotics (for example anthracyclines such as adriamycin, bleomycin, doxorubicin, daunomycin, epirubicin, idarubicin, mitomycin-C, dactinomycin and mithramycin); antimitotic agents (for example vinca alkaloids such as vincristine, vinblastine, vindesine and vinorelbine and taxoids such as taxol and taxotere); and topoisomerase inhibitors (for example epipodophyllotoxins such as etoposide and teniposide, amsacrine, topotecan and camptothecin); and proteosome inhibitors (for example bortezomib [Velcade^{®}]); and the agent anegrilide [Agrylin^{®}]; and the agent alpha-interferon;
- (ii): cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
- (iii): agents which inhibit cancer cell invasion (for example metalloproteinase inhibitors such as marimastat and inhibitors of urokinase plasminogen activator receptor function);
- (iv): inhibitors of growth factor function, for example such inhibitors include growth factor antibodies, growth factor receptor antibodies (for example the anti-erbb2 antibody trastuzumab [Herceptin™] and the anti-erbb1 antibody cetuximab [C225]), farnesyl transferase inhibitors, tyrosine kinase inhibitors and serine/threonine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis (2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family, for example inhibitors or phosphotidylinositol 3-kinase (PI3K) and for example inhibitors of mitogen activated protein kinase (MEK1/2) and for example inhibitors of protein kinase B (PKB/Akt), for example inhibitors of Src tyrosine kinase family and/or Abelson (Abl) tyrosine kinase family such as AZD0530 and dasatinib (BMS-354825) and imatinib mesylate (Gleevec™); and any agents that modify STAT signalling;
- (v): antiangiogenic agents such as those which inhibit the effects of vascular endothelial growth factor, (for example the anti-vascular endothelial cell growth factor antibody bevacizumab [Avastin™], compounds such as those disclosed in International Patent Applications WO 97/22596, WO 97/30035, WO 97/32856 and WO 98/13354) and compounds that work by other mechanisms (for example linomide, inhibitors of integrin α vβ3 function and angiostatin);
- (vi): vascular damaging agents such as Combretastatin A4 and compounds disclosed in International Patent Applications WO 99/02166, WO 00/40529, WO 00/41669, WO 01/92224, WO 02/04434 and WO 02/08213;
- (vii): antisense therapies, for example those which are directed to the targets listed above, such as ISIS 2503, an anti-ras antisense;
- (viii): gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy;
- (ix): immunotherapy approaches, including for example ex-vivo and in-vivo approaches to increase the immunogenicity of patient tumor cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumor cell lines and approaches using anti-idiotypic antibodies and approaches using the immunomodulatory drugs thalidomide and lenalidomide [Revlimid^{®}]; and
- (x): other treatment regimes including: dexamethasone, proteasome inhibitors (including bortezomib), isotretinoin (13-cis retinoic acid), thalidomide, revemid, Rituxamab, ALIMTA, Cephalon's kinase inhibitors CEP-701 and CEP-2563, anti-Trk or anti-NGF monoclonal antibodies, targeted radiation therapy with 1311-metaiodobenzylguanidine (1311-MIBG), anti-G(D2) monoclonal antibody therapy with or without granulocyte- macrophage colony-stimulating factor (GM-CSF) following chemotherapy.

Such conjoint treatment may be achieved by way of the simultaneous, sequential or separate dosing of the individual components of the treatment. Such combination products employ the compounds of this invention, or pharmaceutically acceptable salts thereof, within the dosage range described hereinbefore and the other pharmaceutically-active agent within its approved dosage range.

In addition to its use in therapeutic medicine, compounds of Formula (I) and pharmaceutically acceptable salts thereof are also useful as pharmacological tools in the development and standardization of *in vitro* and *in vivo* test systems for the evaluation of the effects of inhibitors of JAK2 in laboratory animals such as cats, dogs, rabbits, monkeys, rats and mice, as part of the search for new therapeutic agents.

In any of the above-mentioned pharmaceutical composition, process, method, use, medicament, and manufacturing features of the instant invention, any of the alternate embodiments of the compounds of the invention described herein also apply.

In one aspect, the inhibition of JAK activity particularly refers to the inhibition of JAK2 activity.

### Process

If not commercially available, the necessary starting materials for the procedures such as those described herein may be made by procedures which are selected from standard organic chemical techniques, techniques which are analogous to the synthesis of known, structurally similar compounds, or techniques which are analogous to the described procedure or the procedures described in the Examples.

It is noted that many of the starting materials for synthetic methods as described herein are commercially available and/or widely reported in the scientific literature, or could be made from commercially available compounds using adaptations of processes reported in the scientific literature. The reader is further referred to Advanced Organic Chemistry, 5th Edition, by Jerry March and Michael Smith, published by John Wiley & Sons 2001, for general guidance on reaction conditions and reagents.

It will also be appreciated that in some of the reactions mentioned herein it may be necessary/desirable to protect any sensitive groups in compounds. The instances where protection is necessary or desirable are known to those skilled in the art, as are suitable methods for such protection. Conventional protecting groups may be used in accordance with standard practice (for illustration see T.W. Greene, Protective Groups in Organic Synthesis, published by John Wiley and Sons, 1991) and as described hereinabove.

Compounds of Formula (I) may be prepared in a variety of ways. The Processes and Scheme shown below illustrate some methods for synthesizing compounds of Formula (I) and intermediates which may be used for the synthesis of compounds of Formula (I) (wherein **Ring A**, **X**, **R¹**, **R²**, **R³**, and **R⁴**, unless otherwise defined, are as defined hereinabove). Where a particular solvent or reagent is shown in a Scheme or referred to in the accompanying text, it is to be understood that the chemist of ordinary skill in the art will be able to modify that solvent or reagent as necessary. The Processes and Scheme are not intended to present an exhaustive list of methods for preparing the compounds of Formula (I); rather, additional techniques of which the skilled chemist is aware may be also be used for the compounds' synthesis. The claims are not intended to be limited to the structures shown in the Processes and Scheme.

The skilled chemist will be able to use and adapt the information contained and referenced within the above references, and accompanying Examples therein and also the Examples and Scheme herein, to obtain necessary starting materials and products.

In one aspect, compounds of Formula (I), or pharmaceutically acceptable salts thereof, may be prepared by:
1) Process A - reacting a pyrimidine of Formula (A): 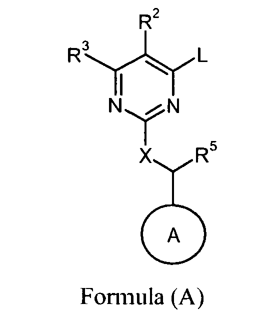 with a compound of Formula (B): 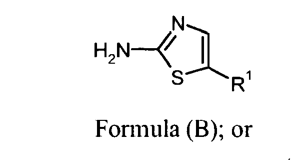
2) Process B - reacting a compound of Formula (C): 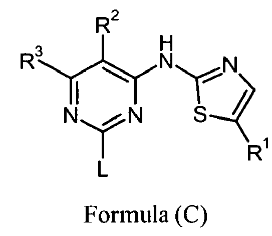 with a compound of Formula (D): 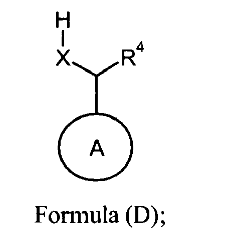 and thereafter if necessary:
   - i): converting a compound of Formula (I) into another compound of Formula (I);
   - ii): removing any protecting groups; and/or
   - iii): forming a pharmaceutically acceptable salt,
   wherein L in each occurrence may be the same or different, and is a leaving group as described hereinabove.

Process A - Compounds of Formula (A) and compounds of Formula (B) may be reacted together in the presence of a suitable solvent, examples of which include ketones such as acetone, alcohols such as ethanol and butanol, and aromatic hydrocarbons such as toluene and N-methyl pyrrolid-2-one. Such reaction may advantageously occur in the presence of a suitablebase, examples of which include, but are not limited to, inorganic bases such as cesium carbonate, potassium carbonateor; and organic bases such astriethylamine, and diisopropylethylamine The reaction is advantageously performed at a temperature in a range from 0°C to reflux.

In another aspect, compounds of Formula (A) and compounds of Formula (B) may be reacted together under standard Buchwald conditions (for example see J. Am. Chem. Soc., 118, 7215; J. Am. Chem. Soc., 119, 8451; J. Org. Chem., 62, 1568 and 6066), with a suitable base. Examples of suitable bases include inorganic bases such as cesium carbonate, and organic bases such as potassium *t-*butoxide. Such a reaction may be advantageously occur in the presence of palladium acetate and appropriate ligands such as BINAP. Solvents suitable for such a reaction include aromatic solvents such as toluene, benzene, or xylene.

Process B - Compounds of Formula (D) and compounds of Formula (B) may be reacted together under conditions similar to those described for the reaction of compounds of Formula (A) with compounds of Formula (B).

The compound of Formula (C) may be prepared according to Scheme 1:

### Scheme 1

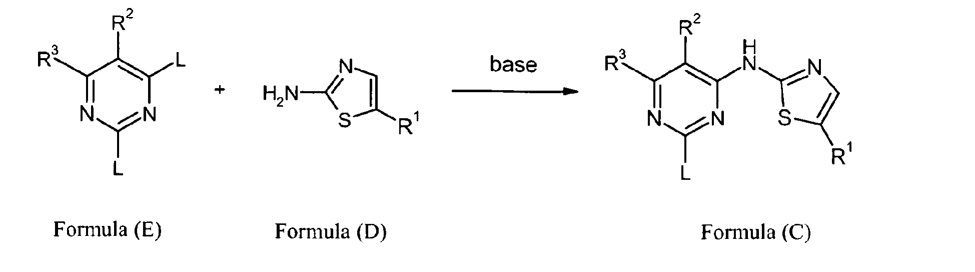

wherein L in each occurrence may be the same or different, and is a leaving group as described hereinabove

### Examples

The invention will now be further described with reference to the following illustrative Examples in which, unless stated otherwise:
- (i): temperatures are given in degrees Celsius (°C); operations are carried out at room temperature or ambient temperature, that is, in a range of 18-25 °C;
- (ii): organic solutions were dried over anhydrous magnesium sulfate unless other wise stated; evaporation of organic solvent was carried out using a rotary evaporator under reduced pressure (4.5 - 30 mmHg) with a bath temperature of up to 60 °C;
- (iii): chromatography means flash chromatography on silica gel; thin layer chromatography (TLC) was carried out on silica gel plates;
- (iv): in general, the course of reactions was followed by TLC or liquid chromatography/mass spectroscopy and reaction times are given for illustration only;
- (v): final products have satisfactory proton nuclear magnetic resonance (NMR) spectra and/or mass spectra data;
- (vi): yields are given for illustration only and are not necessarily those which can be obtained by diligent process development; preparations were repeated if more material was required;
- (vii): when given, NMR data is in the form of delta values for major diagnostic protons, given in part per million (ppm) relative to tetramethylsilane (TMS) as an internal standard, determined at 300 MHz in DMSO-d₆ unless otherwise stated;
- (viii): chemical symbols have their usual meanings;
- (ix): solvent ratio was given in volume : volume (v/v) terms.
- (x): "ISCO" refers to normal phase flash column chromatography using pre-packed silica gel cartridges (12 g, 40 g etc.), used according to the manufacturer's instructions, obtained from ISCO, Inc, 4700 Superior Street Lincoln, NE, USA.
- (xi): A "Gilson column" refers to a YMC-AQC18 reverse phase HPLC Column with dimension 20 mm/100 and 50 mm/250 in H₂O/MeCN with 0.1% TFA as mobile phase unless otherwise stated and used according to the manufacturer's instructions, obtained from Gilson, Inc. 3000 Parmenter Street, Middleton, WI 53562-0027, U.S.A.
- (xii): "Biotage" refers to normal phase flash column chromatography using pre-packed silica gel cartridges (12g, 40g, 80 g etc.), used according to the manufacturer's instructions, obtained from Biotage Inc, 1725 Discovery Drive Charlotteville, Virginia 22911, USA.
- (xiii): "SFC (super critical fluid chromatography)" refers to Analytical SFC (ASC-1000 Analytical SFC System with Diode Array Detector) and/or Preparative SFC (APS- 1000 AutoPrep Preparative SFC),used according to the manufacturer's instruction, obtained from SFC Mettler Toledo AutoChem, Inc. 7075 Samuel Morse Drive Columbia MD 21046, U.S.A.
- (xiv): Parr Hydrogenator or Parr shaker type hydrogenators are systems for treating chemicals with hydrogen in the presence of a catalyst at pressures up to 5 atmospheres (60 psi) and temperatures to 80 °C.
- (xv): the following abbreviations have been used:

| | |
|---|---|
| BINAP | 2,2'-bis(diphenylphosphino)-1,1'-binapthyl |
| Boc₂O | di-*tert*-butyl-dicarbonate |
| DCM | dichloromethane |
| DIPEA | N, N-diisopropylethylamine |
| DMF | *N,N*-dimethylformamide |
| DMAP | 4-dimethylaminopyridine |
| DMSO | dimethylsulfoxide |
| dppf | 1,1'-Bis(diphenylphosphino)ferrocene |
| EtOAc | ethyl acetate |
| Et₂O | diethyl ether |
| GC | gas chromatography |
| HPLC | high-performance liquid chromatography |
| LCMS | liquid chromatography/mass spectroscopy |
| Pd₂(dba)₃ | tris(dibenzylideneacetone)dipalladium (0) |
| THF | tetrahydrofuran |
| TFA | trifluoroacetic acid |

### Intermediate 1

### 2,5-Dichloro-N-(5-methyl-1,3-thiazol-2-yl)pyrimidin-4-amine

To a flask containing 2,4,5-trichloropyrimidine (351 mg, 1.91 mmol) and 5-methyl-1,3-thiazol-2-amine (218 mg, 1.91 mmol) in EtOH (0.35M), DIPEA (0.67 ml, 3.82 mmol) was added. The reaction mixture was stirred overnight at 25°C. The reaction mixture was concentrated in vacuo giving the title compound as yellow oil (539 mg) pure enough to be used in the next step without any further purification. *m*/*z*: 262.

### Intermediate 2

### Methyl 2-[(2,5-dichloropyrimidin-4-yl)amino]-1,3-thiazole-5-carboxylate

The title compound was prepared via a procedure analogous to that described for the synthesis of **Intermediate 1** using 2,4,5-trichloropyrimidine and methyl 2-amino-1,3-thiazole-5-carboxylate. *m*/*z*: 306.

### Intermediate 3

### 5-Fluoropyridine-2-carbonitrile

2-Bromo-5-fluoropyridine (93.0 g, 528 mmol), Zn dust (8.29 g, 127 mmol), zinc cyanide (40.3 g, 343 mmol), 1,1'-bis(diphenylphosphino)ferrocene (11.7 g, 21.1 mmol) and Pd₂dba₃ (9.68 g, 10.6 mmol) in anhydrous DMA (300 ml) was heated at 95 °C for 3 hours. After cooled to room temperature, brine (100 ml) and ether (500 ml) was added. The solid formed was removed by filtration and washed with ether (300 ml). The organic layer was separated, washed with brine (200 ml) and dried over sodium sulfate, and concentrated. After removal of solvent, the resulted residue was purified by column chromatography (hexane-DCM = 1:1) to give the title compound as a white solid (49 g, 72%). ¹H NMR (400 MHz) δ 8.82 (d, 1H), 8.21 (dd, 1H), 8.05 (dd, 1H).

### Intermediate 4

### N-(1-(5-Fluoropyridin-2-yl)vinyl)acetamide

A solution of MeMgBr (170.3 ml, 510.98 mmol) in ether was diluted with 170 ml of anhydrous THF and cooled to 0 °C. 5-Fluoropyridine-2-carbonitrile (**Intermediate 3**, 53.6 g, 425.82 mmol) in THF (170 ml) was added dropwise. The reaction was stirred at 0 °C for 30 minutes, then diluted with DCM (170 ml). Acetic anhydride (48.3 ml, 510.98 mmol) in DCM (100 ml) was added dropwise at 0 °C. After addition, the reaction was warmed to room temperature and stirred at room temperature for 8 hours. Saturated sodium bicarbonate solution (50 ml) was added and extracted with EtOAc (2 x 200 ml). The combined organic was dried over sodium sulfate. After removal of solvent, the resulted residue was purified by column chromatography (hexane : EtOAc = 2.5 : 1) to give the title compound as a white solid (26.6 g, 35%). ¹H NMR (400 MHz) δ 9.37 (s, 1H), 8.57 (d, *J* = 2.8 Hz, 1H), 7.81 (m, 2H), 6.01 (s, 1H), 5.52 (s, 1H), 2.08 (s, 3H). MS: Calculated: 180; Found: [M+H]⁺ 181.

### Intermediate 5

### N-[(1S)-1-(5-Fluoropyridin-2-yl)ethyl]acetamide

To a solution of *N*-(1-(5-fluoropyridin-2-yl)vinyl)acetamide (**Intermediate 4**, 11.0 g, 61.1 mmol) in MeOH (120 ml) under N₂ was added (+)-1,2-bis((2S, 5S)-2,5-diethylphospholano) benzene (cyclooctadiene)rhodium(I)trifluoromethanesulfonate (0.441 g, 0.611 mmol). The solution was transferred to a high pressure bomb and charged 150 psi H₂. The reaction stirred at room temperature and maintained inside pressure between 120-150 psi for 7 hours. The solvent was removed and the resulted residue was purified by column chromatography (EtOAc) to give the title compound as a white solid (9.8 g, 88%). NMR (400 MHz) 8.49 (d, *J*= 2.4 Hz, 1H), 8.32 (d, *J* = 7.6 Hz, 1H), 7.66 (m, 1H), 7.39 (dd, *J* = 4.4 and 8.8 Hz, 1H), 4.95 (m, 1H), 1.85 (s, 3H), 1.34 (d, *J* = 7.2 Hz, 3H). MS: Calculated: 182; Found: [M+H]⁺ 183. Enantiomeric excess determined by HPLC (Chiralpak IA; 70:30 CO₂/MeOH), 95.3% ee.

### Intermediate 6

### tert-Butyl [(1S)-1-(5-fluoropvridin-2-yl)ethyl]carbamate

A solution of *N*-[(1*S*)-1-(5-fluoropyridin-2-yl)ethyl]acetamide (**Intermediate 5**, 11.0 g, 60.37 mmol), DMAP (1.48 g, 12.07 mmol) and di-*tert*-butyl-dicarbonate (26.35 g, 120.7 mmol) in THF (100 ml) was stirred at 50 °C for 20 hours. After cooled to room temperature, lithium hydroxide monohydrate (5.19 g, 123.8 mmol) and water (100 ml) were added. The reaction was stirred at room temperature for 5 hours and diluted with ether (200 ml). The organic layer was separated, washed with brine (100 ml), and dried over sodium sulfate. After removal of solvent, the resulted residue was purified by column chromatography (hexane-EtOAc = 5 : 1) to give the title compound as a pale yellow oil (13.6 g, 94%). NMR (400 MHz) 8.46 (d, *J* = 2.8 Hz, 1H), 7.69 (m, 1H), 7.35-7.41 (m, 2H), 4.67 (m, 1H), 1.37 (s, 9H), 1.32 (d, *J* = 7.2 Hz, 3H). MS: Calculated: 240; Found: [M+H]⁺ 241.

### Intermediate 7

### [(1S)-1-(5-Fluoropyridin-2-yl)ethyl]amine

To a solution of *tert*-Butyl [(1*S*)-1-(5-fluoropyridin-2-yl)ethyl]carbamate (**Intermediate 6**, 12.8 g, 53.3 mmol) in DCM (100 ml) was added HCl/dioxane solution (107 ml, 4 N, 428 mmol). The reaction was stirred at room temperature for 3 hours. The solvent was removed and 50 ml of saturated sodium bicarbonate was added. The resulting aqueous solution was extracted with ether (6 x 400 ml), dried over sodium sulfate and concentrated to give the title compound (7.30 g, 98%) as pale yellow oil. ¹H NMR (400 MHz) 8.44 (d, 1H), 7.66 (m, 1H), 7.53 (m, 1H), 4.01 (q, 1H), 1.94 (b, 2H), 1.26 (d, 3H). MS: Calculated: 140; Found: [M+H]⁺ 141.

### Intermediate 8

### 5-Fluoropyrimidine-2-carbonitrile

A 10 ml microwave vial was charged with 2-chloro-5-fluoropyrimidine (2.0 g, 15.09 mmol), Pd₂(dba)₃ (0.549 g, 0.6 mmol), dppf (0.67 g, 1.21 mmol), zinc cyanide (1.15 g, 9.81 mmol), and zinc dust (0.237 mg, 3.62 mmol). The flask was evacuated and backfilled with N₂, and anhydrous dimethylacetamide. The vial was mounted onto a Personal Chemistry microwave reactor and heated at 100 °C for 10 hours. The reaction mixture was diluted with EtOAc and then washed with brine three times. The organic layer was obtained and evaporated to dryness. The dried residue was purified by silica gel chromatography (By ISCO Combiflash with gradient EtOAc and hexanes) to afford the title compound as a creamy solid (1.50 g, 80%). GC-MS: 123 (M); ¹H NMR (CDCl₃) δ 8.80 (s, 2H).

### Intermediate 9

### N-(1-(5-Fluoropyrimidin-2-yl)vinyl)acetamide

5-Fluoropyrimidine-2-carbonitrile (**Intermediate 8**, 1.0 g, 8.1 mmol) in THF (10 ml) was added a solution of MeMgBr (3.3 ml, 9.75 mmol) in ether drop wise at 0 °C. After addition, the reaction was warmed to room temperature, stirred at room temperature for 1 hour and then diluted with DCM (10 ml). Acetic anhydride (1.23 ml, 13.0 mmol) was added in one portion. The reaction was stirred at room temperature for 1 hour and 40 °C for 1 hour. Saturated sodium bicarbonate solution (10 ml) was added and extracted with EtOAc (2x20 ml). The combined organic was dried over sodium sulfate. After removal of solvent, the resulted residue was purified by column chromatography (hexane : EtOAc = 2.5 : 1) to give the title compound as a white solid (0.38 g, 26%). ¹H NMR (400 MHz) 9.34 (s, 1H), 8.95 (s, 2H), 6.25 (s, 1H), 6.03 (s, 1H), 2.11 (s, 3H). MS: Calculated: 181; Found: [M+H]⁺ 182.

### Intermediate 10

### N-[(1S)-1-(5-Fluoropyrimidin-2-yl)ethyl]acetamide

*N*-(1-(5-Fluoropyrimidin-2-yl)vinyl)acetamide (**Intermediate 9**, 0.10 g, 0.55 mmol) in MeOH (5 ml) under N₂ was added (+)-1,2-bis((2*S*, 5*S*)-2,5-diethylphospholano)benzene (cyclooctadiene)rhodium(I)trifluoromethanesulfonate (0.04 g, 0.0055 mmol). The solution was transferred to a high pressure bomb and charged 150 psi H₂. The reaction was stirred at room temperature for 4 hours. The solvent was removed and the resulted residue was purified by column chromatography (EtOAc) to give the title compound as a white solid (0.096 g, 95%). ¹H NMR (400 MHz) 8.84 (d, *J* = 0.8 Hz, 2H), 8.34 (d, *J* = 7.6 Hz, 1H), 5.00 (m, 1H), 1.84 (s, 3H), 1.37 (d, *J* = 6.8 Hz, 3H). MS: Calculated: 183; Found: [M+H]⁺ 184. Enantiomeric excess determined by HPLC (Chiralpak IA; 95:5 CO₂/MeOH), >99% ee.

### Intermediate 11

### tert-Butyl[(1S)-1-(5-Fluoropyrimidin-2-yl)ethyl]carbamate

A solution of *N*-[(1*S*)-1-(5-fluoropyrimidin-2-yl)ethyl]acetamide (**Intermediate 10**, 0.20 g, 1.09 mmol), DMAP (0.027 g, 0.22 mmol) and di-tert-butyl-dicarbonate (0.60 g, 2.73 mmol) in THF (10 ml) was stirred at 50 °C for 40 hours. After cooling to room temperature, lithium hydroxide monohydrate (0.094 g, 2.24 mmol) and water (10 ml) was added. The reaction was stirred at room temperature for 9 hours. Ether (30 ml) was added, organic layer was separated, washed with brine (20 ml) and dried over sodium sulfate. After removal of solvent, the resulted residue was purified by column chromatography (Hex : EtOAc = 5 : 1) to give the title compound as a pale yellow oil (0.21 g, 80%). ¹H NMR (400 MHz) 8.84 (s, 2H), 7.24 (d, *J* = 7.6 Hz, 1H), 4.74 (m, 1H), 1.35 (s, 12H). MS: Calculated: 241; Found: [M+H]⁺ 242.

### Intermediate 12

### (1S)-1-(5-Fluoropyrimidin-2-yl)ethanamine hydrochloride

To a solution of *tert*-Butyl [(1*S*)-1-(5-Fluoropyrimidin-2-yl)ethyl]carbamate (**Intermediate 11**, 0.21 g, 0.87 mmol) in DCM (5 ml) was added HCl (1.3 ml, 5.2 mmol) in dioxane. The reaction was stirred at room temperature for 3 hours. The solvent was removed give the title compound as white solid (quantitative). MS: Calculated: 141; Found: [M+H]⁺ 142.

**Intermediate 12** may also be prepared from 5-fluoropyrimidine-2-carbonitrile (**Intermediate 8**):

### Intermediate 12 (alternate synthetic route)

### (1S)-1-(5-Fluoropyrimidin-2-yl)ethanamine hydrochloride

To a solution of *N*-[(1*S*)-1-(5-fluoropyrimidin-2-yl)ethyl]-(2*R*)-methylpropane-2-sulfinamide (**Intermediate 15**, 655 mg, 2.67 mmol) in dry dioxane (20 ml) was added HCl (3.4 ml, 13.3 mmol) in dioxane. The reaction was stirred at room temperature for 3 hours. The solvent was removed to give the title compound as white solid (quantitative). MS: Calculated: 141; Found: [M+H]⁺ 142.

### Intermediate 13

### 5-Fluoropyrimidine-2-carbaldehyde

To a solution of 5-fluoropyrimidine-2-carbonitrile (**Intermediate 8**, 1.0 g, 8.1 mmol) in anhydrous THF at -78°C was added a solution of DIBAL-H (8.1 mL) over a period of 20 minutes. The resulting mixture was stirred at this temperature for 2hours whereupon MeOH was added. The solution was allowed to warm to room temperature whereupon a solution of conc. HCl was added. The resulting mixture was stirred for 2 hours at ambient temperature and the aqueous layer was washed with EtOAc (3x). The combined organic extracts were washed with brine and dried (MgSO₄). Evaporation of the solvent afforded the titled compound (780 mg, 76%). MS: [M+H]⁺ 127.

### Intermediate 14

### N-[(1Z and/or E)-(5-Fluoropyrimidin-2-yl)methylene]-2-(R)-methylpropane-2-sulfinamide

To a solution of 5-fluoropyrimidine-2-carbaldehyde (**Intermediate 13**, 1.55 g, 12.3 mmol) in anhydrous DCM at room temperature were added 2-(*R*)-methylpropane-2-sulfinamide (1.79 g, 14.7 mmol) and anhydrous copper(II)sulphate (1.96 g, 12.28 mmol). The resulting mixture was stirred at this temperature for 24 hours, the solid was filtered under vacuum, washed with DCM (3x) and evaporation of the solvents afforded a yellow oil. The resulted residue was purified by column chromatography (Hex : EtOAc = 3 : 1) to give the title compound (1.94 g, 69%). 1H NMR (300 MHz, DMSO-d6) δ ppm 9.13 (s, 2 H) 8.47 (s, 1 H) 0.99 (s, 9 H). MS:[M+H]⁺ 232.

### Intermediate 15

### N-[(1S)-1-(5-Fluoropyrimidin-2-yl)ethyl]-(2R)-methylpropane-2-sulfinamide

To a solution of *N*-[(1*Z* and/or *E*)-(5-fluoropyrimidin-2-yl)methylene]-2-(*R*)-methylpropane-2-sulfinamide (**Intermediate 14**, 1.94 g, 8.5 mmol) in anhydrous THF at -20°C was added slowly a solution of MeMgBr (9.3 mL g, 9.3 mmol). The resulting mixture was stirred at this temperature for 3 hours whereupon it partitioned between H₂O and EtOAc. The aqueous layer was extracted with EtOAc (3x) washed with brine, dried (MgSO₄). Evaporation of the solvents under reduced pressure under vacuum afforded yellow oil. The resulted residue was purified by column chromatography (100% EtOAc) to give the title compound (660 mg, 50%). 1H NMR (300 MHz, DMSO-d6) δ ppm 8.89 (s, 2 H) 5.53 (d, 1 H) 4.43 - 4.65 (m, 1 H) 1.46 (d, 3 H) 1.11 (s, 9 H). MS:[M+H]⁺ 246.

### Intermediate 16

### 4-(2,6-Dichloro-5-fluoropyrimidin-4-yl)morpholine

A round-bottom flask was charged 2,4,6-trichloro-5-fluoropyrimidine (see PCT Pub. No. WO05/049033, 2.0 g, 10 mmol), in ethanol (100ml), and was cooled to -20C. Morpholine (0.95 g, 11 mmo) in EtOH (20 ml) was added drop-wise to the reaction mixture in the course of I hour. The reaction was stirred at -20°C for 30 minutes and at room temperature overnight. Solvent was removed *in vacuo* and the residue was partitioned between DCM and H₂O. Organic phase was concentrated down, and the residue was recrystallized from EtOH to afford the title compound (1.75 g, 86%). LC-MS, 252 (M+1). ¹H NMR (DMSO) δ 6.76 (s, 1H), 3.69 (m, 8H).

### Intermediate 17

### 4-Chloro-5- fluoro-N-[(1S)-1-(5-fluoropyrimidin-2-yl)ethyl]-6-morpholin-4-ylpyrimidin-2-amine

A round-bottom flask was charged with 4-(2,6-dichloro-5-fluoropyrimidin-4-yl)morpholine (**Intermediate 16**, 1.45 g, 5.75 mmol), (1*S*)-1-(5-fluoropyrimidin-2-yl)ethanamine hydrochloride (**Intermediate 12**, 1.23 g, 5.75 mmol), DIPEA (4.0 ml, 23 mmol) in *n*-BuOH (20ml). The reaction was heated at 130°C overnight. Evaporation of the solvents followed by purification (ISCO Combiflash with gradient EtOAc/hexanes with 1% Et₃N) afforded the title compound 345 mg. LC-MS, 357 (M+1). ¹H NMR (DMSO) δ 8.80 (s, 2H), 7.65 (br, 1H), 4.90 (br,, 1H), 3.46 (br, 8H), 1.43 (d, 3H).

### Intermediate 18

### 2,4-Dichloro-6-(4-methyl-piperazin-1-yl)-pyrimidine

A round-bottom flask was charged 2,4,6-trichloropyrimidine (0.92 g, 5 mmol) and DIPEA (1.74 ml, 10 mmol) in EtOH (15ml) was cooled to -20°C. 4-Methylpiperazine (0.55 mL, 5 mmol) in EtOH (5 ml) was added drop-wise. The reaction was stirred at -20°C for 30 minutes and then at 0°C for 1 hour. The mixture was warmed to room temperature and stirred overnight at this temperature. Evaporation of the solvents *in vacuo* gave a residue, which was purified on silica gel chromatography to afford the title compound (0.8 g, 81%). LC-MS, 248 (M+1).

### Intermediate 19

### 4-Chloro-N-[(1S)-1-(5-fluoropyridin-2-yl)ethyl]-6-(4-methylpiperazin-1-yl)pyrimidin-2-amine

A round-bottom flask was charged with 2,4-dichloro-6-(4-methyl-piperazin-1-yl)-pyrimidine **(Intermediate 18,** 0.50 g, 2 mmol), (1*S*)-1-(5-fluoropyridin-2-yl)ethanamine hydrochloride (0.42 g, 2 mmol), DIPEA (1.4 ml, 8 mmol) in *n-*BuOH (8ml). The reaction was heated at reflux overnight. Evaporation of the solvents followed by column chromatography purification (ISCO Combiflash with gradient DCM and MeOH with 1% Et₃N). A white gum was obtained (0.14g, 20%). LC-MS, 351 (M+1).

### Intermediate 20

### 4-(2,6-Dichloropyrimidin-4-yl)morpholine

The title compound was prepared via a procedure analogous to that described for the synthesis of **Intermediate 18** using 2,4,6-trichloropyrimidine and morpholine. LC-MS: 235 (M+1).

### Intermediate 21

### 4-Chloro-N-[(1S)-1-(5-fluoropyrimidin-2-yl)ethyl]-6-morpholin-4-ylpyrimidin-2-amine

4-(2,6-Dichloropyrimidin-4-yl)morpholine **(Intermediate 20)** and (1*S*)-1-(5-fluoropyrimidin-2-yl)ethanamine hydrochloride **(Intermediate 12)** were reacted using a procedure analogous to that described for the synthesis of **Intermediate 19,** providing the title compound. *m*/*z:* 339.

### Intermediate 22

### 4-Chloro-N-[(1S)-1-(5-fluoropyridin-2-yl)ethyl]-6-morpholin-4-ylpyrimidin-2-amine

4-(2,6-Dichloropyrimidin-4-yl)morpholine **(Intermediate 20)** and [(1*S*)-1-(5-fluoropyridin-2-yl)ethyl]amine **(Intermediate 7)** were reacted using a procedure analogous to that described for the synthesis of **Intermediate 19,** providing the title compound. *m*/*z*: 338.

### Intermediate 23

### 2.6-Dichloro-N-(5-methyl-1,3-thiazol-2-yl)pyrimidin-4-amine

To a flask containing 2,4,6-trichloropyrimidine (4.81 g, 26.2 mmol) and 5-methyl-1,3-thiazol-2-amine (3.0 g, 26.2 mmol) in THF (0.2M), was added NaH (60% w/w in mineral oil, 1.6 g, 39.4 mmol) with caution at 0°C. The reaction mixture was stirred for 2 hours at 25°C. The reaction mixture was diluted with H₂O and the product was collected via filtration. *m*/*z*: 261.

### Intermediate 24

### 6-Chloro-N²-[(1S)-1-(5-fluoropyridin-2yl)ethyl]-N⁴-(5-methyl-1,3-thiazol-2-yl)pyrimidine-2,4-diamine

To a solution of 2,6-dichloro*-N-*(5-methyl-1,3-thiazol-2-yl)pyrimidin-4-amine **(Intermediate 23,** 346 mg, 1.33 mmol) in *n-*BuOH (5 ml) was added DIPEA (0.5 ml, 2.66 mmol) and (S)-1-(5-fluoropyridin-2-yl)ethanamine hydrochloride (0.22 g, 1.33 mmol) and the resulting solution was heated to reflux for 12 hours. The mixture was partitioned between EtOAc and H₂O, the organic layer was washed with brine and dried. The solvents were removed under reduced pressure to give an oil which was purified by column chromatography (30%→50% EtOAc/Hex) to afford the title compound (1.8 g). *m*/*z:* 366.

### Intermediate 25

### 2-Amino-1,3-thiazole-5-carbonitrile

To a solution of a mixture of 2-(E and Z)-3-methoxyacrylonitrile (0.18 mmol, 1.51 mL) in MeCN (3 ml) was added at 0°C and with caution bromine (~1 mL, 0.18 mmol). The resulting mixture was stirred at this temperature for 30 minutes, whereupon cold water (12 ml) was added. After vigorous stirring for 1 hr, NaOAc·3H₂O (16 mmol) was added and stirred for 15 minutes. Thiourea (19.8 mmol) was added and the solution was stirred for 2 hours at 0C, NaOAc·3H₂O (10.8 mmol) was added and the resulting mixture was heated to 60°C for 1 hr. The mixture was allowed to cool to room temperature and stirred overnight at this temperature. 10N NaOH (aq) was added slowly into the reaction mixture until the pH-4. The title compound was collected by filtration after being washed with cold H₂O. LC-MS: 126 (M+1). ¹H NMR (DMSO-d6) δ 8.10 (s, 2H), 7.81 (s, 1H).

### Intermediate 26

### 2-[(2,6-Dichloropyrimidin-4-yl)amino]-1,3-thiazole-5-carbonitrile

2-Amino-1,3-thiazole-5-carbonitrile (Intermediate 25) and 2,4,6-trichloropyrimidine were reacted using a procedure analogous to that described for the synthesis of **Intermediate 23,** providing the title compound. *m*/*z*: 272.

### Intermediate 27

### 2-[(6-Chloro-2-{[(1S)-1-(5-fluoropyridin-2-yl)ethyl]amino}pyrimidin-4-yl)amino]-1,3-thiazole-5-carbonitrile

2-[(2,6-Dichloropyrimidin-4-yl)amino]-1,3-thiazole-5-carbonitrile **(Intermediate 26)** and (S)-1-(5-fluoropyridin-2-yl)ethanamine hydrochloride **(Intermediate 7)** were reacted using a procedure analogous to that described for the synthesis of **Intermediate 24,** providing the title compound. *m*/*z*: 371.

### Intermediate 28

### 2-[(6-Chloro-2-{[(1S)-1-(5-fluoropyrimidin-2-yl)ethyl]amino}pyrimidin-4-yl)amino]-1,3-thiazole-5-carbonitrile

2-[(2,6-dichloropyrimidin-4-yl)amino]-1,3-thiazole-5-carbonitrile **(Intermediate 26)** and (S)-1-(5-fluoropyrimidin-2-yl)ethanamine hydrochloride (Intermediate 12) were reacted using a procedure analogous to that described for the synthesis of **Intermediate 27,** providing the title compound. *m*/*z*: 372

### Intermediate 28

### (S)-4-chloro-N-(1-(5-fluoropyrimidin-2-yl)ethyl)-6-methoxypyrimidin-2-amine

2,4-dichloro-6-methoxypyrimidine **(Intermediate 29)** and (1*S*)-1-(5-Fluoropyrimidin-2-yl)ethanamine hydrochloride **(Intermediate 12)** were reacted using a procedure analogous to that described for the synthesis of **Intermediate 17,** providing the title compound. LCMS: 284 [M+H]⁺

### Intermediate 29

### 2,4-Dichloro-6-methoxypyrimidine

2,4,6-trichloropyrimidine (1.82g, 10 mmol) in THF (40 mL) was cooled at -40°C. NaOMe (20 ml, 0.5M in MeOH) was added to the reaction drop wise via syringe. The reaction mixture was stirred at -40°C for additional 40 mins and subsequently warmed up to room temperature over 1h. The precipitate was filtered off, and the volatiles were evaporated under reduced pressure to afford the title compound that was used in the next step without any further purification LCMS: 180 [M+H]⁺

### Example 1

### 5-Chloro-N²-[(1S)-1-(4-fluorophenyl)ethyl]-N⁴-(5-methyl-1,3-thiazol-2-yl)pyrimidine-2,4-diamine

A microwave reaction vessel was charged with 2,5-dichloro-*N-*(5-methyl-1,3-thiazol-2-yl)pyrimidin-4-amine **(Intermediate 1,** 800 mg, 3.06 mmol), (1*S*)-1-(4-fluorophenyl)ethanamine (426 mg, 3.06 mmol) and DIPEA (1.07 mL, 6.12 mmol). Anhydrous *n-*butanol (0.5M) was then added and the tube was sealed and heated at 180°C for 10800 seconds in a microwave reactor. The reaction mixture was concentrated in vacuo giving a brown oil. Purification by Gilson (10-50% MeCN/H₂O, 35 min) gave a yellow oil (221 mg). This material was then re-purified by SFC giving the title compound as a white solid (17 mg). ¹H NMR (300 MHz, DMSO-d6) δ: 7.91 - 8.03 (s, 2 H) 7.37 (dd, 2 H) 6.99 - 7.12 (m, 2 H) 5.19 (br.s, 1H) 2.26 (s, 3 H) 1.42 (d, 3 H). *m*/*z*: 364.

### Example 2

### Methyl 2-[(5-chloro-2-{[(1S)-1-(4-fluorophenyl)ethyl]amino}pyrimidin-4-yl)amino]-1,3-thiazole-5-carboxylate

Methyl 2-[(2,5-dichloropyrimidin-4-yl)amino]-1,3-thiazole-5-carboxylate **(Intermediate 2)** and (1*S*)-1-(4-fluorophenyl)ethanamine were reacted using a procedure analogous to that described for the synthesis of **Example 1,** providing the title compound. ¹H NMR (300 MHz, DMSO-d6) δ ppm 8.16 (s, 1 H) 7.46 (dd, 2 H) 7.12 (m, 2 H) 5.30 (br.s, 1 H) 3.86 (s, 3 H) 1.49 (d, 3 H). /z: 408.

### Example 3

### 5-Chloro-N²-[(1S)-1-(5-fluoropyridin-2-yl)ethyl]-N⁴-(5-methyl-1,3-thiazol-2-yl)pyrimidine-2,4-diamine

2,5-Dichloro-*N-*(5-methyl-1,3-thiazol-2-yl)pyrimidin-4-amine **(Intermediate 1)** and [(1*S*)-1-(5-fluoropyridin-2-yl)ethyl]amine **(Intermediate 7)** were reacted using a procedure analogous to that described for the synthesis of **Example 1,** providing the title compound. ¹H NMR (300 MHz, DMSO-d6) δ ppm 2.38 - 2.51 (m, 3 H) 4.77 - 5.06 (m, 1 H) 7.23 - 7.52 (m, 2 H) 7.57 - 7.77 (m, 1 H) 8.48 (s, 1 H). *m*/*z:* 365.

### Example 4

### 5-Fluoro-N²-[(1S)-1-(5-fluoropyrimidin-2-yl)ethyl]-N⁴-(5-methyl-1,3-thiazol-2-yl)-6-morpholin-4-ylpyrimidine-2,4-diamine

A microwave reaction vessel was charged with 4-chloro-5-fluoro-*N-*[(1*S*)-1-(5-fluoropyrimidin-2-yl)ethyl]-6-morpholin-4-ylpyrimidin-2-amine **(Intermediate 17,** 72 mg, 0.2 mmol), 5-methyl-1,3-thiazol-2-amine (28 mg, 0.22 mmol), Pd₂(dba)₃ (18 mg, 0.02 mmol), BINAP (25 mg, 0.04 mmol), CS₂CO₃ (228 mg, 0.7 mmol) in dioxane. The reaction was degassed and was heated at 95°C for 6 hours. Solvent was removed and the residue was partitioned between DCM and H₂O. The dichloromethane layer was concentrated down and was purified by silica gel chromatography twice (by ISCO Combiflash with gradient MeOH and DCM) to afford the title compound as a light pale solid (47 mg, 52%). LC-MS, 435 (M+1). ¹H NMR (DMSO) δ 8.50 (s, 2H), 7.02 (s, 1H), 5.21(m, 1H), 3.63 (m, 4H), 3.52 (m, 4H), 2.32 (s, 3H), 1.54 (d, 3H).

### Example 5

### N²-[(S)-1-(5-Fluoro-pyridin-2-yl)-ethyl]-6-(4-methyl-piperazin-1-yl)-N⁴-(5-methyl-thiazol-2-yl)-pyrimidine-2,4-diamine

4-Chloro -*N-*[(1*S*)-1-(5-fluoropyridin-2-yl)ethyl]-6-(4-methyl-piperazi-1-yl)-pyrimidin-2-amine **(Intermediate 19)** and 5-methyl-1,3-thiazol-2-amine were reacted using a procedure analogous to that described for the synthesis of **Example 4,** providing the title compound. ¹H NMR (CDCl₃) δ 8.26 (s, 1H), 7.44 (m, 1H), 7.37 (m, 1H), 7.00 (s, 1H), 6.21 (s, 1H), 4.99 (m, 1H), 4.27 (m, 211), 3.35 (m, 2H), 3.12 (m, 2H), 2.90 m, 2H), 2.74 (s, 3H), 2.22 (s, 3H), 1.41 (d, 3H). *m*/*z* 429.

### Example 6

### N²-[(1S)-1-(5-Fluoropyrimidin-2-yl)ethyl]-N⁴-(5-methyl-1,3-thiazol-2-yl)-6-morpholin-4-ylpyrimidine-2,4-diamine

4-Chloro-*N-*[(1*S*)-1-(5-fluoropyrimidin-2-yl)ethyl]-6-morpholin-4-ylpyrimidin-2-amine **(Intermediate 21)** and 5-methyl-1,3-thiazol-2-amine were reacted using a procedure analogous to that described for the synthesis of **Example 4,** providing the title compound. ¹H NMR (300 MHz, CHLOROFORM-d) δ ppm 1.66 (d, 3 H) 2.55 (s, 3 H) 3.48 - 3.69 (m, 4 H) 3.73 - 3.93 (m, 4 H) 5.23 - 5.41 (m, 1 H) 5.71 (s, 1 H) 7.01 (s, 1H) 8.77 (s, 2 H) 9.42 (s, 1H). *m*/*z:* 417

### Example 7

### N²-[(1S)-1-(5-Fluoropyridin-2-yl)ethyl]-N⁴-(5-methyl-1,3-thiazol-2-yl)-6-morpholin-4-ylpyrimidine-2,4-diamine

4-Chloro-*N-*[(1*S*)-1-(5-fluoropyridin-2-yl)ethyl]-6-morpholin-4-ylpyrimidin-2-amine **(Intermediate 22)** and 5-methyl-1,3-thiazol-2-amine were reacted using a procedure analogous to that described for the synthesis of **Example 1,** providing the title compound. ¹H NMR (300 MHz, DMSO-d6) δ ppm 1.42 (s, 3H) 2.35 (s, 3H) 3.22 - 3.85 (m, 8H) 4.96 - 5.33 (m, 2H) 5.50 (s, 1 H) 7.06 (s, 1H) 7.45 (dd,1 H) 7.56 - 7.75 (m, 1H) 8.47 (s, 1H). *m*/*z* 416.

### Example 7 (alternate synthetic route)

### N²-[(1S)-1-(5-Fluoropyridin-2-yl)ethyl]-N⁴-(5-methyl-1,3-thiazol-2-yl)-6-morpholin-4-ylpyrimidine-2,4-diamine

To a solution of 6-chloro*-N*²-[(1,*S*)-1-(5-fluoropyridin-2-yl)ethyl]*-N*⁴-(5-methyl-1,3-thiazol-2-yl)pyrimidine-2,4-diamine **(Intermediate 24,** 200 mg) in i-AmOH (0.5 ml) was added morpholine (5 ml) and the resulting solution was heated to reflux for 12 hours. The mixture was partitioned between EtOAc and H₂O, the organic layer was washed with brine and dried. The solvents were removed under reduced pressure to give an oil Purification by Gilson (20-95% MeCN/H₂O, 35 min) afforded the title compound. ¹H NMR (300 MHz, DMSO-d6) δ ppm 1.42 (s, 3 H) 2.35 (s, 3 H) 3.22 - 3.85 (m, 8 H) 4.96 - 5.33 (m, 2 H) 5.50 (s, 1 H) 7.06 (s, 1 H) 7.45 (dd,1 H) 7.56 - 7.75 (m, 1 H) 8.47 (s, 1 H). *m*/*z:* 416.

### Example 8

### 2-[(2-{(1,S)-1-(5-Fluoropyridin-2-yl)ethyl]amino}-6-morpholin-4-ylpyrimidin-4-yl)amino]-1,3-thiazole-5-carbonitrile

2-[(6-Chloro-2-{[(1*S*)-1-(5-fluoropyridin-2-yl)ethyl]amino}pyrimidin-4-yl)amino]-1,3-thiazole-5-carbonitrile **(Intermediate 27)** and morpholine were reacted using a procedure analogous to that described for the synthesis of **Example .7,** providing the title compound. ¹H NMR (300 MHz, CHLOROFORM-d) δ ppm 1.62 (s, 3 H) 3.27- 3.97 (m, 8 H) 5.14 - 5.49 (m, 1 H) 5.51 (s, 1H) 7.59 - 7.74 (m, 2 H) 7.80 (s, 1 H) 8.48 (s, 1 H). *m*/*z*: 427.

### Example 9

### 2-[(2-{[(1S)-1-(5-Fluoropyrimidin-2-yl)ethyl]amino}-6-morpholin-4-ylpyrimidin-4-yl)amino]-1,3-thiazole-5-carbonitrile

2-[(6-Chloro-2- {[(1*S*)-1-(5-fluoropyrimidin-2-yl)ethyl]amino} pyrimidin-4-yl)amino]-1,3 -thiazole-5-carbonitrile **(Intermediate 28)** and morpholine were reacted using a procedure analogous to that described for the synthesis of **Example 7,** providing the title compound. ¹H NMR (300 MHz, CHLOROFORM-d) δ ppm 1.61 (d, 3 H) 3.05 - 4.23 (m, 8 H) 4.71 - 4.91 (m, 1 H) 5.18 (s, 1 H) 7.92 (s, 1 H) 8.57 (s, 2 H). *m*/*z:* 428.

### Example 10

### (S)-N²-1-(5-Fluoropyrimidin-2-yl)ethyl)-6-methoxy-N⁴-(5-methylthiazol-2-yl)pyrimidine-2.4-diamine

(S)-4-Chloro-N-(1-(5-fluoropyrimidin-2-yl)ethyl)-6-methoxypyrimidin-2-amine **(Intermediate 28)** and 5-methyl-1,3-thiazol-2-amine were reacted using a procedure analogous to that described for the synthesis of **Example 4,** providing the title compound. ¹H NMR (400 MHz, DMSO-d6) δ ppm 10.91 (s, 1 H) 8.86 (s, 2 H) 7.37 (s, 1 H) 6.98 (s, 1 H) 5.38 (s, 1 H) 5.18(m, 1H) 3.81 (s, 3H) 2.28 (s, 3 H) 1.56 (d, 3 H). LCMS: 362 [M+H]⁺

## Claims

1. A compound of Formula (I): 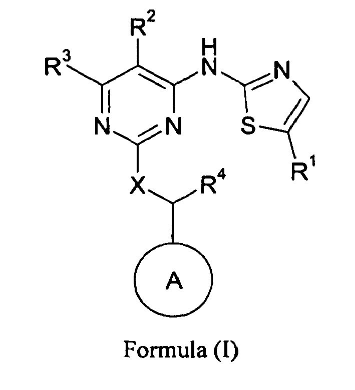 or a pharmaceutically acceptable salt thereof, wherein:
**Ring A** is selected from 4-fluorophenyl, 5-fluoropyridin-2-yl, and 5-fluoropyrimidin-2-yl;
**X** is -NH-
**R¹** is selected from -CN, methyl, and -C(O)₂Me;
**R²** is selected from H, fluoro, and chloro;
**R³** is selected from H, methoxy, 4-methylpiperazin-1-yl, morpholin-4-yl, and piperazin-1yl; and
**R⁴** is methyl.

2. A compound according to claim 1 wherein the compound is selected from
5-Chloro*-N*²-[(1*S*)-1-(4-fluorophenyl)ethyl]*-N*⁴-(5-methyl-1,3-thiazol-2-yl)pyrimidine-2,4-diamine;
Methyl 2-[(5-chloro-2-{[(1*S*)-1-(4-fluorophenyl)ethyl]amino}pyrimidin-4-yl)amino]-1,3-thiazole-5-carboxylate;
5-Chloro*-N*²-[(1*S*)-1-(5-fluoropyridin-2-yl)ethyl]*-N*⁴-(5-methyl-1,3-thiazol-2-yl)pyrimidine-2,4-diamine;
5-Fluoro*-N*²-[(1*S*)-1-(5-fluoropyrimidin-2-yl)ethyl]*-N*⁴-(5-methyl-1,3-thiazol-2-yl)-6-morpholin-4-ylpyrimidine-2,4-diamine;
*N*²-[(*S*)-1-(5-Fluoro-pyridin-2-yl)-ethyl]-6-(4-methyl-piperazin-l-yl)-N⁴-(5-methyl-thiazol-2-yl)-pyrimidine-2,4-diamine;
*N*²-[(1,*S*)-1-(5-Fluoropyrimidin-2-yl)ethyl]*-N*⁴-(5-methyl-1,3-thiazol-2-yl)-6-morpholin-4-ylpyrimidine-2,4-diamine;
*N*²-[(1,*S*)-1-(5-Fluoropyridin-2-yl)ethyl]*-N*⁴-(5-methyl-1,3-thiazol-2-yl)-6-morpholin-4-ylpyrimidine-2,4-diamine;
2-[(2-{[(1,*S*)-1-(5-Fluoropyridin-2-yl)ethyl]amino}-6-morpholin-4-ylpyrimidin-4-yl)amino]-1,3-thiazole-5-carbonitrile;
2-[(2-{[(1*S*-1-(5-Fluoropyrimidin-2-yl)ethyl]amino}-6-morpholin-4-ylpyrimidin-4-yl)amino]-1,3-thiazole-5-carbonitrile; and
(S)*-N*²-(1-(5-Fluoropyrimidin-2-yl)ethyl)-6-methoxy*-N*⁴-(5-methylthiazol-2-yl)pyrimidine-2,4-diamine.

3. A compound of Formula (I), or a pharmaceutically acceptable salt thereof, as claimed in claim 1 or 2, for use as a medicament.

4. The use of a compound of Formula (I), or a pharmaceutically acceptable salt thereof, as claimed in claim 1 or 2, in the manufacture of a medicament for use in treating myeloproliferative disorders, myelodysplastic syndrome, and cancer, in a warm-blooded animal such as man.

5. The use of a compound of Formula (I), or a pharmaceutically acceptable salt thereof, as claimed in claim 1 or 2, in the manufacture of a medicament for treating myeloproliferative disorders, myelodysplastic syndrome and cancers (solid and hematologic tumors), fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acromegaly, acute and chronic inflammation, bone diseases, and ocular diseases with retinal vessel proliferation, in a warm-blooded animal such as man.

6. The use of a compound of Formula (I), or a pharmaceutically acceptable salt thereof, as claimed in claim 1 or 2, in the manufacture of a medicament for treating chronic myeloid leukemia, polycythemia vera, essential thrombocythemia, myeloid metaplasia with myelofibrosis, idiopathic myelofibrosis, chronic myelomonocytic leukemia and hypereosinophilic syndrome, myelodysplastic syndromes and cancers selected from oesophageal cancer, myeloma, hepatocellular, pancreatic, cervical cancer, Ewings sarcoma, neuroblastoma, Kaposi's sarcoma, ovarian cancer, breast cancer, colorectal cancer, prostate cancer, bladder cancer, melanoma, lung cancer - non small cell lung cancer (NSCLC), and small cell lung cancer (SCLC), gastric cancer, head and neck cancer, mesothelioma, renal cancer, lymphoma and leukaemia, in a warm-blooded animal such as man.

7. The use of a compound of Formula (I), or a pharmaceutically acceptable salt thereof, as claimed in claim 1 or 2, in the manufacture of a medicament for the production of an anti-proliferative effect, in a warm-blooded animal such as man.

8. The use of a compound of Formula (I), or a pharmaceutically acceptable salt thereof, as claimed in claim 1 or 2, in the manufacture of a medicament for the treatment of cancer.

9. A compound of Formula (I), or a pharmaceutically acceptable salt thereof, as claimed in claim 1 or 2, for use in treating myeloproliferative disorders, myelodysplastic syndrome, and cancer, in a warm-blooded animal such as man.

10. A compound of Formula (I), or a pharmaceutically acceptable salt thereof, as claimed in claim 1 or 2, for use in treating myeloproliferative disorders, myelodysplastic syndrome, and cancers (solid and hematologic tumors), fibroproliferative and differentiative disorders, psoriasis, rheumatoid arthritis, Kaposi's sarcoma, haemangioma, acute and chronic nephropathies, atheroma, atherosclerosis, arterial restenosis, autoimmune diseases, acromegaly, acute and chronic inflammation, bone diseases, and ocular diseases with retinal vessel proliferation, in a warm-blooded animal such as man.

11. A compound of Formula (I), or a pharmaceutically acceptable salt thereof, as claimed in claim 1 or 2, for use in treating chronic myeloid leukemia, polycythemia vera, essential thrombocythemia, myeloid metaplasia with myelofibrosis, idiopathic myelofibrosis, chronic myelomonocytic leukemia and hypereosinophilic syndrome, myelodysplastic syndromes and cancers selected from oesophageal cancer, myeloma, hepatocellular, pancreatic, cervical cancer, Ewings sarcoma, neuroblastoma, Kaposi's sarcoma, ovarian cancer, breast cancer, colorectal cancer, prostate cancer, bladder cancer, melanoma, lung cancer - non small cell lung cancer (NSCLC), and small cell lung cancer (SCLC), gastric cancer, head and neck cancer, mesothelioma, renal cancer, lymphoma and leukaemia, in a warm-blooded animal such as man.

12. A compound of Formula (I), or a pharmaceutically acceptable salt thereof, as claimed in claim 1 or 2, for use in the production of an anti-proliferative effect in a warm-blooded animal such as man.

13. A compound of Formula (I), or a pharmaceutically acceptable salt thereof, as claimed in claim 1 or 2, for use in the treatment of cancer in a warm-blooded animal such as man.

14. A pharmaceutical composition comprising a compound of Formula (I), or a pharmaceutically acceptable salt thereof, as claimed in claim 1 or 2, and at least one pharmaceutically acceptable carrier, diluent, or excipient.

15. A process for preparing a compound of Formula (I), or a pharmaceutically acceptable salt thereof, as claimed in claim 1 or 2, said process comprising reacting a compound of Formula (A): 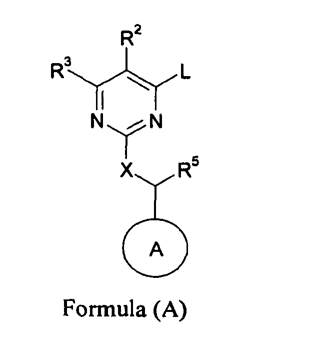 with a compound of Formula (B): 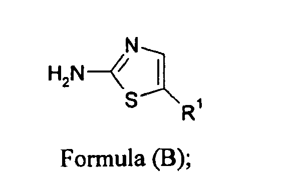 and thereafter if necessary:
i) converting a compound of Formula (I) into another compound of Formula (I);
ii) removing any protecting groups; and/or
iii) forming a pharmaceutically acceptable salt,
wherein L is a leaving group.

16. A process for preparing a compound of Formula (I), or a pharmaceutically acceptable salt thereof, as claimed in claim 1 or 2, said process comprising reacting a compound of Formula (C): 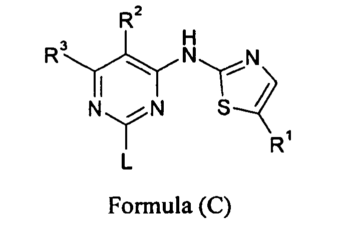 with a compound of Formula (D): 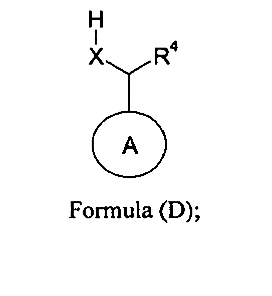 and thereafter if appropriate:
i. converting a compound of Formula (I) into another compound of Formula (I);
ii. removing any protecting groups; and/or
iii. forming a pharmaceutically acceptable salt,
wherein L is a leaving group.

## Patentansprüche

1. Verbindung der Formel (I): 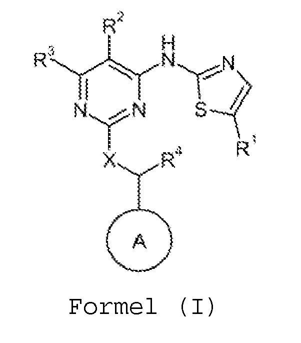 oder ein pharmazeutisch annehmbares Salz davon, wobei:
Ring A unter 4-Fluorphenyl, 5-Fluorpyridin-2-yl und 5-Fluorpyrimidin-2-yl ausgewählt ist;
X für -NH- steht;
R¹ unter -CN, Methyl und -C(O) ₂Me ausgewählt ist;
R² unter H, Fluor und Chlor ausgewählt ist;
R³ unter H, Methoxy, 4-Methylpiperazin-1-yl,
Morpholin-4-yl und Piperazin-1-yl ausgewählt ist und
R⁴ für Methyl steht.

2. Verbindung nach Anspruch 1, ausgewählt unter
5-Chlor-*N*²-[(1*S*)-1-(4-fluorphenyl)ethyl]-*N*⁴- (5-methyl-1,3-thiazol-2-yl)pyrimidin-2,4-diamin;
2- [(5-Chlor-2-{[(1*S*)-1-(4-fluorphenyl)ethyl] - amino}pyrimidin-4-yl)amino]-1,3-thiazol-5-carbonsäuremethylester;
5-Chlor*-N*²- [(1*S*)-1-(5-fluorpyridin-2-yl)ethyl] *-N*⁴-(5-methyl-1,3-thiazol-2-yl)pyrimidin-2,4-diamin; 5-Fluor*-N*²-[(1*S*)-1-(5-fluorpyrimidin-2-yl)ethyl]-*N*⁴-(5-methyl-1,3-thiazol-2-yl)-6-morpholin-4-yl-pyrimidin-2,4-diamin;
*N*²-[(*S*)-1-(5-Fluorpyridin-2-yl)ethyl]-6-(4-methylpiperazin-1-yl)*-N*⁴-(5-methylthiazol-2-yl)pyrimidin-2,4-diamin;
*N²-*[(1*S*)-1-(5-Fluorpyrimidin-2-yl)ethyl] *-N*⁴ -(5-methyl-1,3-thiazol-2-yl)-6-morpholin-4-yl-pyrimidin-2,4-diamin;
*N²-*[(1*S*)-1-(5-Fluorpyridin-2-yl)ethyl]*-N*⁴-(5-methyl-1,3-thiazol-2-yl)-6-morpholin-4-yl-pyrimidin-2,4-diamin;
2-[(2-{[(1*S*)-1-(5-Fluorpyridin-2-yl)ethyl]amino}-6-morpholin-4-ylpyrimidin-4-yl)amino]-1,3-thiazol-5-carbonitril;
2-[(2-{[(1*S*)-1-(5-Fluorpyrimidin-2-yl)ethyl] - amino}-6-morpholin-4-ylpyrimidin-4-yl)amino]-1,3-thiazol-5-carbonitril und (*S*)*-N*²-(1-(5-Fluorpyrimidin-2-yl)ethyl)-6-methoxy-*N*⁴-(5-methylthiazol-2-yl)pyrimidin-2,4-diamin.

3. Verbindung der Formel (I) oder ein pharmazeutisch annehmbares Salz davon nach Anspruch 1 oder 2 zur Verwendung als Arzneimittel.

4. Verwendung einer Verbindung der Formel (I) oder eines pharmazeutisch annehmbaren Salzes davon nach Anspruch 1 oder 2 bei der Herstellung eines Arzneimittels zur Verwendung bei der Behandlung von myeloproliferativen Störungen, myelodysplastischem Syndrom und Krebs bei einem Warmblüter wie dem Menschen.

5. Verwendung einer Verbindung der Formel (I) oder eines pharmazeutisch annehmbaren Salzes davon nach Anspruch 1 oder 2 bei der Herstellung eines Arzneimittels zur Behandlung von myeloproliferativen Störungen, myelodysplastischem Syndrom und Krebserkrankungen (soliden und hämatologischen Tumoren), fibroproliferativen und differentiativen Störungen, Psoriasis, rheumatoider Arthritis, Kaposi-Sarkom, Hämangiom, akuten und chronischen Nephropathien, Atherom, Atherosklerose, arterieller Restenose, Autoimmunerkrankungen, Akromegalie, akuter und chronischer Entzündung, Knochenerkrankungen und Augenerkrankungen mit Netzgefäßhautproliferation bei einem Warmblüter wie dem Menschen.

6. Verwendung einer Verbindung der Formel (I) oder eines pharmazeutisch annehmbaren Salzes davon nach Anspruch 1 oder 2 bei der Herstellung eines Arzneimittels zur Behandlung von chronischer myeloischer Leukämie, Polycythemia vera, essentieller Thrombozythämie, myeloischer Metaplasie mit Myelofibrose, idiopathischer Myelofibrose, chronischer myelomonozytärer Leukämie und hypereosinophilem Syndrom, myelodysplastischen Syndromen und Speiseröhrenkrebs, Myelom, Leberzellkrebs, Bauchspeicheldrüsenkrebs, Zervixkrebs, Ewing-Sarkom, Neuroblastom, Kaposi-Sarkom, Eierstockkrebs, Brustkrebs, Kolorektalkrebs, Prostatakrebs, Blasenkrebs, Melanom, Lungenkrebs - nicht-kleinzelligem Lungenkrebs (NSCLC) und kleinzelligem Lungenkrebs (SCLC), Magenkrebs, Kopf- und Halskrebs, Mesotheliom, Nierenkrebs, Lymphom und Leukämie bei einem Warmblüter wie dem Menschen.

7. Verwendung einer Verbindung der Formel (I) oder eines pharmazeutisch annehmbaren Salzes davon nach Anspruch 1 oder 2 bei der Herstellung eines Arzneimittels zur Hervorrufung einer antiproliferativen Wirkung bei einem Warmblüter wie dem Menschen.

8. Verwendung einer Verbindung der Formel (I) oder eines pharmazeutisch annehmbaren Salzes davon nach Anspruch 1 oder 2 bei der Herstellung eines Arzneimittels zur Behandlung von Krebs.

9. Verbindung der Formel (I) oder eines pharmazeutisch annehmbaren Salzes davon nach Anspruch 1 oder 2 zur Verwendung bei der Behandlung von myeloproliferativen Störungen, myelodysplastischem Syndrom und Krebs bei einem Warmblüter wie dem Menschen.

10. Verbindung der Formel (I) oder eines pharmazeutisch annehmbaren Salzes davon nach Anspruch 1 oder 2 zur Verwendung bei der Behandlung von myeloproliferativen Störungen, myelodysplastischem Syndrom und Krebserkrankungen (soliden und hämatologischen Tumoren), fibroproliferativen und differentiativen Störungen, Psoriasis, rheumatoider Arthritis, Kaposi-Sarkom, Hämangiom, akuten und chronischen Nephropathien, Atherom, Atherosklerose, arterieller Restenose, Autoimmunerkrankungen, Akromegalie, akuter und chronischer Entzündung, Knochenerkrankungen und Augenerkrankungen mit Netzgefäßhautproliferation bei einem Warmblüter wie dem Menschen.

11. Verbindung der Formel (I) oder eines pharmazeutisch annehmbaren Salzes davon nach Anspruch 1 oder 2 zur Verwendung bei der Behandlung von chronischer myeloischer Leukämie, Polycythemia vera, essentieller Thrombozythämie, myeloischer Metaplasie mit Myelofibrose, idiopathischer Myelofibrose, chronischer myelomonozytärer Leukämie und hypereosinophilem Syndrom, myelodysplastischen Syndromen und Speiseröhrenkrebs, Myelom, Leberzellkrebs, Bauchspeicheldrüsenkrebs, Zervixkrebs, Ewing-Sarkom, Neuroblastom, Kaposi-Sarkom, Eierstockkrebs, Brustkrebs, Kolorektalkrebs, Prostatakrebs, Blasenkrebs, Melanom, Lungenkrebs - nicht-kleinzelligem Lungenkrebs (NSCLC) und kleinzelligem Lungenkrebs (SCLC), Magenkrebs, Kopf- und Halskrebs, Mesotheliom, Nierenkrebs, Lymphom und Leukämie bei einem Warmblüter wie dem Menschen.

12. Verbindung der Formel (I) oder eines pharmazeutisch annehmbaren Salzes davon nach Anspruch 1 oder 2 zur Verwendung bei der Hervorrufung einer antiproliferativen Wirkung bei einem Warmblüter wie dem Menschen.

13. Verbindung der Formel (I) oder eines pharmazeutisch annehmbaren Salzes davon nach Anspruch 1 oder 2 zur Verwendung bei der Behandlung von Krebs bei einem Warmblüter wie dem Menschen.

14. Pharmazeutische Zusammensetzung, enthaltend eine Verbindung der Formel (I) oder ein pharmazeutisch annehmbares Salz davon nach Anspruch 1 oder 2 und mindestens einen pharmazeutisch annehmbaren Träger, ein pharmazeutisch annehmbares Verdünnungsmittel oder einen pharmazeutisch annehmbaren Hilfsstoff.

15. Verfahren zur Herstellung einer Verbindung der Formel (I) oder eines pharmazeutisch annehmbaren Salzes davon nach Anspruch 1 oder 2, bei dem man eine Verbindung der Formel (A): 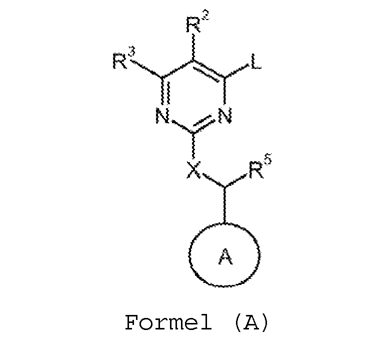 mit einer Verbindung der Formel (B): 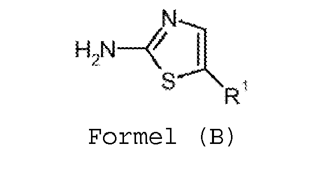 umsetzt und danach gegebenenfalls:
i) eine Verbindung der Formel (I) in eine andere Verbindung der Formel (I) umwandelt;
ii) gegebenenfalls vorhandene Schutzgruppen abspaltet und/oder
iii) ein pharmazeutisch annehmbares Salz bildet, wobei L für eine Abgangsgruppe steht.

16. Verfahren zur Herstellung einer Verbindung der Formel (I) oder eines pharmazeutisch annehmbaren Salzes davon nach Anspruch 1 oder 2, bei dem man eine Verbindung der Formel (C): 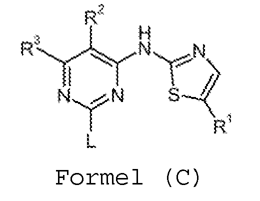 mit einer Verbindung der Formel (D): 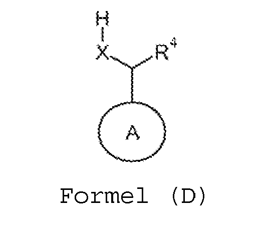 umsetzt und danach gegebenenfalls:
i) eine Verbindung der Formel (I) in eine andere Verbindung der Formel (I) umwandelt;
ii) gegebenenfalls vorhandene Schutzgruppen ab- spaltet und/oder
iii) ein pharmazeutisch annehmbares Salz bildet,
wobei L für eine Abgangsgruppe steht.

## Revendications

1. Composé de Formule (I) : 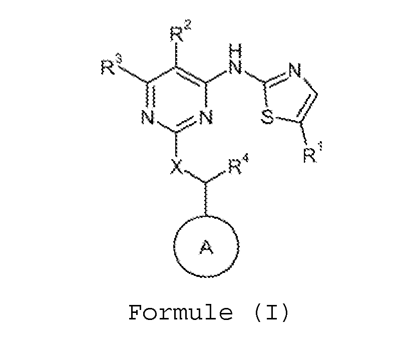 ou un sel pharmaceutiquement acceptable de celui-ci, dans laquelle :
le cycle A est choisi parmi 4-fluorophényle, 5-fluoropyridin-2-yle et 5-fluoropyrimidin-2-yle ;
X est -NH- ;
R¹ est choisi parmi -CN, méthyle et -C(O)₂Me ;
R² est choisi parmi H, fluoro et chloro ;
R³ est choisi parmi H, méthoxy, 4-méthylpipérazin-1-yle, morpholin-4-yle et pipérazin-1-yle ; et
R⁴ est méthyle.

2. Composé selon la revendication 1, dans lequel le composé est choisi parmi
la 5-chloro-N²-[(1S)-1-(4-fluorophényl)éthyl]-N⁴-(5-méthyl-1,3-thiazol-2-yl)pyrimidine-2,4-diamine ;
le 2-[(5-chloro-2-{[(1S)-1-(4-fluorophényl)éthyl]-amino}pyrimidin-4-yl)amino]-l,3-thiazole-5-carboxylate de méthyle ;
la 5-chloro-N²-[(1S)-1-(5-fluoropyridin-2-yl)éthyl]-N⁴-(5-méthyl-1,3-thiazol-2-yl)pyrimidine-2,4-diamine ;
la 5-fluoro-N²- [(1S)-1-(5-fluoropyrimidin-2-yl)éthyl] - N⁴-(5-méthyl-1,3-thiazol-2-yl)-6-morpholin-4-yl-pyrimidine-2,4-diamine ;
la N²-[(S)-1-(5-fluoropyridin-2-yl)éthyl]-6-(4-méthyl-pipérazin-1-yl)-N4-(5-méthylthiazol-2-yl)pyrimidine-2,4-diamine ;
la N²- [(1S)-1-(5-fluoropyrimidin-2-yl)éthyl]-N⁴-(5-méthyl-1,3-thiazol-2-yl)-6-morpholin-4-ylpyrimidine-2,4-diamine ;
la N²-[(1S)-1-(5-fluoropyridin-2-yl)éthyl]-N4-(5-méthyl-1,3-thiazol-2-yl)-6-morpholin-4-ylpyrimidine-2,4-diamine ;
le 2-[(2-{[(1S)-1-(5-fluoropyridin-2-yl)éthyl]amino-6-morpholin-4-ylpyrimidin-4-yl)amino]-1,3-thiazole-5-carbonitrile ;
le 2-[(2-{[(1S)-1-(5-fluoropyrimidin-2-yl)éthyl]amino-6-morpholin-4-ylpyrimidin-4-yl)amino]-1,3-thiazole-5-carbonitrile ; et
la (S)-N²-(1-(5-fluoropyrimidin-2-yl)éthyl)-6-méthoxy-N⁴-(5-méthylthiazol-2-yl)pyrimidine-2,4-diamine.

3. Composé de Formule (I), ou un sel pharmaceutiquement acceptable de celui-ci, selon la revendication 1 ou 2, pour une utilisation comme médicament.

4. Utilisation d'un composé de Formule (I), ou d'un sel pharmaceutiquement acceptable de celui-ci, selon la revendication 1 ou 2, pour la fabrication d'un médicament destiné à une utilisation dans le traitement des troubles myéloprolifératifs, du syndrome myélodysplasique, et du cancer, chez un animal à sang chaud tel que l'homme.

5. Utilisation d'un composé de Formule (I), ou d'un sel pharmaceutiquement acceptable de celui-ci, selon la revendication 1 ou 2, pour la fabrication d'un médicament destiné au traitement des troubles myéloprolifératifs, du syndrome myélodysplasique, et des cancers (tumeurs solides et hématologiques), des troubles de fibroprolifération et de différentiation, du psoriasis, de la polyarthrite rhumatoïde, du sarcome de Kaposi, de l'hémangiome, des néphropathies aiguës et chroniques, de l'athérome, de l'athérosclérose, de la resténose artérielle, des maladies auto-immunes, de l'acromégalie, des inflammations aiguës et chroniques, des maladies osseuses, et des maladies oculaires avec prolifération des vaisseaux rétiniens, chez un animal à sang chaud tel que l'homme.

6. Utilisation d'un composé de Formule (I), ou d'un sel pharmaceutiquement acceptable de celui-ci, selon la revendication 1 ou 2, pour la fabrication d'un médicament destiné au traitement de la leucémie myéloïde chronique, de l'érythrémie, de la thrombocythémie essentielle, de la métaplasie myéloïde avec myélofibrose, de la myélofibrose idiopathique, de la leucémie myélomonocytique chronique et du syndrome d'hyperéosinophilie, des syndromes myélodysplasiques et des cancers choisis parmi le cancer de l'oesophage, les myélomes, le cancer hépatocellulaire, pancréatique, du col de l'utérus, le sarcome d'Ewing, les neuroblastomes, le sarcome de Kaposi, le cancer de l'ovaire, le cancer du sein, le cancer colorectal, le cancer de la prostate, le cancer de la vessie, les mélanomes, le cancer du poumon - le cancer du poumon non à petites cellules (NSCLC), et le cancer du poumon à petites cellules (SCLC), le cancer de l'estomac, le cancer de la tête et du cou, les mésothéliomes, le cancer du rein, les lymphomes et la leucémie, chez un animal à sang chaud tel que l'homme.

7. Utilisation d'un composé de Formule (I), ou d'un sel pharmaceutiquement acceptable de celui-ci, selon la revendication 1 ou 2, pour la fabrication d'un médicament destiné à la production d'un effet antiprolifératif, chez un animal à sang chaud tel que l'homme.

8. Utilisation d'un composé de Formule (I), ou d'un sel pharmaceutiquement acceptable de celui-ci, selon la revendication 1 ou 2, pour la fabrication d'un médicament destiné au traitement du cancer.

9. Composé de Formule (I), ou un sel pharmaceutiquement acceptable de celui-ci, selon la revendication 1 ou 2, pour une utilisation dans le traitement des troubles myéloprolifératifs, du syndrome myélodysplasique, et du cancer, chez un animal à sang chaud tel que l'homme.

10. Composé de Formule (I), ou un sel pharmaceutiquement acceptable de celui-ci, selon la revendication 1 ou 2, pour une utilisation dans le traitement des troubles myéloprolifératifs, du syndrome myélodysplasique, et des cancers (tumeurs solides et hématologiques), des troubles de fibroprolifération et de différentiation, du psoriasis, de la polyarthrite rhumatoïde, du sarcome de Kaposi, de l'hémangiome, des néphropathies aiguës et chroniques, de l'athérome, de l'athérosclérose, de la resténose artérielle, des maladies auto-immunes, de l'acromégalie, des inflammations aiguës et chroniques, des maladies osseuses, et des maladies oculaires avec prolifération des vaisseaux rétiniens, chez un animal à sang chaud tel que l'homme.

11. Composé de Formule (I), ou un sel pharmaceutiquement acceptable de celui-ci, selon la revendication 1 ou 2, pour une utilisation dans le traitement de la leucémie myéloïde chronique, de l'érythrémie, de la thrombocythémie essentielle, de la métaplasie myéloïde avec myélofibrose, de la myélofibrose idiopathique, de la leucémie myélomonocytique chronique et du syndrome d'hyperéosinophilie, des syndromes myélodysplasiques et des cancers choisis parmi le cancer de l'oesophage, les myélomes, le cancer hépatocellulaire, pancréatique, du col de l'utérus, le sarcome d'Ewing, les neuro*blastomes, le sarcome de Kaposi, le cancer de l'ovaire, le cancer du sein, le cancer colorectal, le cancer de la prostate, le cancer de la vessie, les mélanomes, le cancer du poumon - le cancer du poumon non à petites cellules (NSCLC), et le cancer du poumon à petites cellules (SCLC), le cancer de l'estomac, le cancer de la tête et du cou, les mésothéliomes, le cancer du rein, les lymphomes et la leucémie, chez un animal à sang chaud tel que l'homme.

12. Composé de Formule (I), ou un sel pharmaceutiquement acceptable de celui-ci, selon la revendication 1 ou 2, pour une utilisation dans la production d'un effet antiprolifératif, chez un animal à sang chaud tel que l'homme.

13. Composé de Formule (I), ou un sel pharmaceutiquement acceptable de celui-ci, selon la revendication 1 ou 2, pour une utilisation dans le traitement du cancer, chez un animal à sang chaud tel que l'homme.

14. Composition pharmaceutique comprenant un composé de Formule (I), ou un sel pharmaceutiquement acceptable de celui-ci, selon la revendication 1 ou 2, et au moins un véhicule, diluant ou excipient pharmaceutiquement acceptable.

15. Procédé de préparation d'un composé de Formule (I), ou d'un sel pharmaceutiquement acceptable de celui-ci, selon la revendication 1 ou 2, ledit procédé comprenant la réaction d'un composé de Formule (A) : 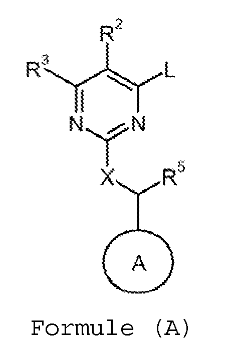 avec un composé de Formule (B) : 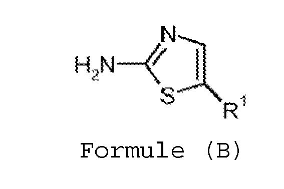 puis, si nécessaire :
i. la conversion d'un composé de Formule (I) en un autre composé de Formule (I) ;
ii. l'élimination de groupements protecteurs quelconques ; et/ou
iii. la formation d'un sel pharmaceutiquement acceptable,
où L est un groupement partant.

16. Procédé de préparation d'un composé de Formule (I), ou d'un sel pharmaceutiquement acceptable de celui-ci, selon la revendication 1 ou 2, ledit procédé comprenant la réaction d'un composé de Formule (C) : 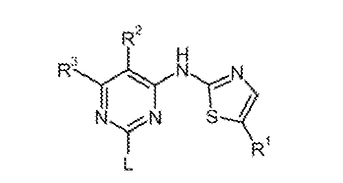 Formule (C) avec un composé de Formule (D) : 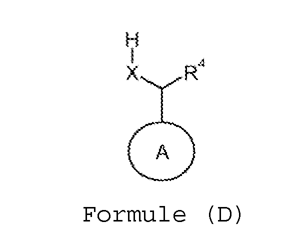 puis, si nécessaire :
i. la conversion d'un composé de Formule (I) en un autre composé de Formule (I) ;
ii. l'élimination de groupements protecteurs quelconques ; et/ou
iii. la formation d'un sel pharmaceutiquement acceptable,
où L est un groupement partant.
